# EUROPEAN PATENT APPLICATION

(11) **EP 2 407 487 A1**
(43) Date of publication of application: **18.01.2012**
(21) Application number: 10169566.6
(22) Date of filing: 14.07.2010
(51) Int. Cl.: C07K 16/32, C07K 16/30

(54) **Multispecific modular antibody**

(71) Applicant: F-Star Biotechnologische Forschungs - und Entwicklungsges. M.B.H., 1230 Wien (AT)
(72) Inventor: Himmler, Gottfried, 2301 Gross-Enzersdorf (AT); Woisetschlaeger, Max, 2380 Perchtoldsdorf (AT)
(74) Representative: Maschio, Antonio

(57) **Abstract**

The invention relates to a modular antibody having at least two specificities to crosslink a glycoepitope and a receptor of the erbB class on a tumor cell, a method of producing such antibody and its use as a therapeutic.

SEQUENCE LISTING

<110>  f-star Biotechnologische Forschungs- und
       ENtwicklungsges.m.b.H.

<120>  Cross-linking modular antibody

<130>  FS013EP

<160>  15

<170>  PatentIn version 3.3

<210>  1
<211>  16
<212>  PRT
<213>  artificial

<220>
<223>  CDR sequence

<400>  1

Arg Ser Ser Gln Ser Ile Val His Ser Asn Gly Asn Thr Tyr Leu Glu
1               5                   10                  15


<210>  2
<211>  7
<212>  PRT
<213>  artificial

<220>
<223>  CDR sequence

<400>  2

Lys Val Ser Asn Arg Phe Ser
1               5


<210>  3
<211>  9
<212>  PRT
<213>  artificial

<220>
<223>  CDR Sequence

<400>  3

Phe Gln Gly Ser His Val Pro Phe Thr
1               5


<210>  4
<211>  5
<212>  PRT
<213>  artificial

<220>
<223>  CDR Sequence

<400>  4

Asp Tyr Tyr Met Tyr
1               5

<210>  5
<211>  17
<212>  PRT
<213>  artificial

<220>
<223>  CDR Sequence

<400>  5

Tyr Ile Ser Asn Gly Gly Gly Ser Ser His Tyr Val Asp Ser Val Lys
1               5                   10                  15

Gly

<210>  6
<211>  10
<212>  PRT
<213>  artificial

<220>
<223>  CDR Sequence

<400>  6

Gly Met Asp Tyr Gly Ala Trp Phe Ala Tyr
1               5                   10

<210>  7
<211>  122
<212>  PRT
<213>  artificial

<220>
<223>  BW835 VH

<400>  7

Glu Val Lys Leu Glu Glu Ser Gly Gly Gly Leu Val Gln Pro Gly Gly
1               5                   10                  15

Ser Met Lys Leu Ser Cys Val Ala Ser Gly Phe Thr Phe Ser Asn Tyr
            20                  25                  30

Trp Met Asn Trp Val Arg Gln Ser Pro Glu Lys Gly Leu Glu Trp Val
        35                  40                  45

Ala Glu Ile Arg Leu Lys Ser Asn Asn Tyr Ala Thr His Tyr Ala Glu
    50                  55                  60

Ser Val Lys Gly Arg Phe Thr Ile Ser Arg Asp Asp Ser Lys Ser Ser
65                  70                  75                  80

Val Tyr Leu Gln Met Asn Asn Leu Arg Ala Glu Asp Thr Gly Ile Tyr
                85                  90                  95

Tyr Cys Ile Arg Glu Thr Val Phe Tyr Tyr Tyr Ala Met Asp Tyr Trp

            100                 105                 110

Gly Gln Gly Thr Thr Val Thr Val Ser Ser
        115                 120

<210> 8
<211> 112
<212> PRT
<213> artificial

<220>
<223> BW835 VL

<400> 8

Asp Ile Val Met Thr Gln Thr Pro Pro Ser Val Pro Val Thr Pro Gly
1               5                   10                  15

Glu Ser Val Ser Ile Ser Cys Arg Ser Ser Gln Ser Leu Leu His Gly
            20                  25                  30

Asp Gly Asn Thr Tyr Leu Tyr Trp Phe Leu Gln Arg Pro Gly Gln Ser
        35                  40                  45

Pro Arg Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Gly Gly Thr Lys Val Glu Ile Lys
            100                 105                 110

<210> 9
<211> 119
<212> PRT
<213> artificial

<220>
<223> VL311 VH

<400> 9

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Tyr Met Tyr Trp Val Arg Gln Ala Pro Glu Lys Arg Leu Glu Trp Val
        35                  40                  45

Ala Tyr Ile Ser Asn Gly Gly Gly Ser Ser His Tyr Val Asp Ser Val

50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ala Glu Asp Thr Ala Leu Tyr His Cys
                85                  90                  95

Ala Arg Gly Met Asp Tyr Gly Ala Trp Phe Ala Tyr Trp Gly Gln Gly
            100                 105                 110

Thr Leu Val Thr Val Ser Ser
        115

<210>  10
<211>  112
<212>  PRT
<213>  artificial

<220>
<223>  VL311 VL

<400>  10

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Ile Val His Ser
            20                  25                  30

Asn Gly Asn Thr Tyr Leu Glu Trp Tyr Leu Gln Lys Pro Gly Gln Ser
        35                  40                  45

Pro Gln Leu Leu Ile Ser Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Gly
                85                  90                  95

Ser His Val Pro Phe Thr Phe Gly Gln Gly Thr Lys Leu Glu Ile Lys
            100                 105                 110

<210>  11
<211>  223
<212>  PRT
<213>  artificial

<220>
<223>  Fcab H561-4

<400>  11

Thr Cys Pro Pro Cys Pro Ala Pro Glu Leu Leu Gly Gly Pro Ser Val

1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Lys Phe Asn Trp Tyr Val Asp Gly Val Glu Val His Asn Ala Lys
    50                  55                  60

Thr Lys Pro Arg Glu Glu Gln Tyr Asn Ser Thr Tyr Arg Val Val Ser
65                  70                  75                  80

Val Leu Thr Val Leu His Gln Asp Trp Leu Asn Gly Lys Glu Tyr Lys
                85                  90                  95

Cys Lys Val Ser Asn Lys Ala Leu Pro Ala Pro Ile Glu Lys Thr Ile
            100                 105                 110

Ser Lys Ala Lys Gly Gln Pro Arg Glu Pro Gln Val Tyr Thr Leu Pro
        115                 120                 125

Pro Ser Arg Asp Glu Leu Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Val Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220

<210>  12
<211>  10
<212>  PRT
<213>  artificial

<220>
<223>  Fcwt AB loop sequence

<400>  12

Asp Glu Leu Thr Lys Asn Gln Val Ser Leu
1               5                   10

<210>  13
<211>  11
<212>  PRT
<213>  artificial

<220>
<223>  Fcwt EF loop sequence

<400>  13

Thr Val Asp Lys Ser Arg Trp Gln Gln Gly Asn
1               5                   10


<210>  14
<211>  10
<212>  PRT
<213>  artificial

<220>
<223>  Fcab AB loop sequence

<400>  14

Asp Glu Phe Phe Thr Tyr Trp Val Ser Leu
1               5                   10


<210>  15
<211>  11
<212>  PRT
<213>  artificial

<220>
<223>  Fcab EF loop sequence

<400>  15

Thr Val Asp Arg Arg Arg Trp Thr Ala Gly Asn
1               5                   10

## Description

The invention relates to a multispecific modular antibody.

### Background

Monoclonal antibodies have been widely used as therapeutic binding agents. The basic antibody structure will be explained here using as example an intact IgG1 immunoglobulin.

Two identical heavy (H) and two identical light (L) chains combine to form the Y-shaped antibody molecule. The heavy chains each have four domains. The amino terminal variable domains (VH) are at the tips of the Y. These are followed by three constant domains: CH1, CH2, and the carboxy-terminal CH3, at the base of the Y's stem. A short stretch, the switch, connects the heavy chain variable and constant regions. The hinge connects CH2 and CH3 (the Fc fragment) to the remainder of the antibody (the Fab fragments). One Fc and two identical Fab fragments can be produced by proteolytic cleavage of the hinge in an intact antibody molecule. The light chains are constructed of two domains, variable (VL) and constant (CL), separated by a switch.

Disulfide bonds in the hinge region connect the two heavy chains. The light chains are coupled to the heavy chains by additional disulfide bonds. Asn-linked carbohydrate moieties are attached at different positions in constant domains depending on the class of immunoglobulin. For IgG1 two disulfide bonds in the hinge region, between Cys235 and Cys238 pairs, unite the two heavy chains. The light chains are coupled to the heavy chains by two additional disulfide bonds, between Cys229s in the CH1 domains and Cys214s in the CL domains. Carbohydrate moieties are attached to Asn306 of each CH2, generating a pronounced bulge in the stem of the Y.

These features have profound functional consequences. The variable regions of both the heavy and light chains (VH) and (VL) lie at the "tips" of the Y, where they are positioned to react with antigen. This tip of the molecule is the side on which the N-terminus of the amino acid sequence is located. The stem of the Y projects in a way to efficiently mediate effector functions such as the activation of complement and interaction with Fc receptors, or ADCC and ADCP. Its CH2 and CH3 domains bulge to facilitate interaction with effector proteins. The C-terminus of the amino acid sequence is located on the opposite side of the tip, which can be termed "bottom" of the Y.

Two types of light chain, termed lambda (λ) and kappa (k), are found in antibodies. A given immunoglobulin either has k chains or λ chains, never one of each. No functional difference has been found between antibodies having λ or k light chains.

Each domain in an antibody molecule has a similar structure of two beta sheets packed tightly against each other in a compressed antiparallel beta barrel. This conserved structure is termed the immunoglobulin fold. The immunoglobulin fold of constant domains contains a 3-stranded sheet packed against a 4-stranded sheet. The fold is stabilized by hydrogen bonding between the beta strands of each sheet, by hydrophobic bonding between residues of opposite sheets in the interior, and by a disulfide bond between the sheets. The 3-stranded sheet comprises strands C, F, and G, and the 4-stranded sheet has strands A, B, E, and D. The letters A through G denote the sequential positions of the beta strands along the amino acid sequence of the immunoglobulin fold.

The fold of variable domains has 9 beta strands arranged in two sheets of 4 and 5 strands. The 5-stranded sheet is structurally homologous to the 3-stranded sheet of constant domains, but contains the extra strands C' and C". The remainder of the strands (A, B, C, D, E, F, G) have the same topology and similar structure as their counterparts in constant domain immunoglobulin folds. A disulfide bond links strands B and F in opposite sheets, as in constant domains.

The variable domains of both light and heavy immunoglobulin chains contain three hypervariable loops, or complementarity-determining regions (CDRs). The three CDRs of a V domain (CDR1, CDR2, CDR3) cluster at one end of the beta barrel. The CDRs are loops that connect beta strands B-C, C'-C", and F-G of the immunoglobulin fold. The residues in the CDRs vary from one immunoglobulin molecule to the next, imparting antigen specificity to each antibody.

The VL and VH domains at the tips of antibody molecules are closely packed such that the 6 CDRs (3 on each domain) cooperate in constructing a surface (or cavity) for antigen-specific binding. The natural antigen binding site of an antibody thus is composed of the loops which connect strands B-C, C'-C", and F-G of the light chain variable domain and strands B-C, C'-C", and F-G of the heavy chain variable domain.

The loops which are not CDR-loops in a native immunoglobulin, or not part of the antigen-binding pocket as determined by the CDR loops and optionally adjacent loops within the CDR loop region, do not have antigen binding or epitope binding specificity, but contribute to the correct folding of the entire immunoglobulin molecule and/or its effector or other functions and are therefore called structural loops for the purpose of this invention.

Prior art documents show that the immunoglobulin-like scaffold has been employed so far for the purpose of manipulating the existing antigen binding site, thereby introducing novel binding properties. In most cases the CDR regions have been engineered for antigen binding, in other words, in the case of the immunoglobulin fold, only the natural antigen binding site has been modified in order to change its binding affinity or specificity. A vast body of literature exists which describes different formats of such manipulated immunoglobulins, frequently expressed in the form of single-chain Fv fragments (scFv) or Fab fragments, either displayed on the surface of phage particles or solubly expressed in various prokaryotic or eukaryotic expression systems.

WO06/072620A1 describes a method of engineering an immunoglobulin which comprises a modification in a structural loop region to obtain new antigen binding sites. This method is broadly applicable to immunoglobulins and may be used to produce a library of immunoglobulins targeting a variety of antigens. A CH3 library has been shown to be useful for selecting specific binders to an antigen.

WO08/003103A2 describes the panning of a CH3, CH1 or CL library on a synthetic peptide, representing a mimotope of the CD20 antigen.

Immunoglobulins based on full length IgG1 have been used to target tumor antigens including those associated with aberrant glycosylation of a tumor cell, such as a glycosylation bearing antigens, which are highly expressed in many types of epithelial cancers. Among them are blood group antigen related glycepitopes, such as Lewis x-, Lewis b- and Lewis y-structures, including sialylated Lewis x-structures. Other carbohydrate antigens are Globo H-structures, KH1, Tn antigen, TF antigen, such as the Thomsen-Friedenreich (TF)-disaccharide (Galβ1-3GalNAc-), β-galactoside sequences of several cell surface structures (e.g. (Galβ1-4GlcNAc), the alpha-1,3-galactosyl epitope (Elektrophoresis (1999), 20:362; Curr. Pharmaceutical Design (2000), 6:485, Neoplasma (1996), 43:285) and carbohydrate structures of Mucins, CD44 including its splice variants, especially CD44v6, glycolipids and glycosphingolipids, such as Gg3, Gb3, GD3, GD2, Gb5, Gm1, Gm2, sialyltetraosylceramide.

Monoclonal antibody BW835 defines a carbohydrate epitope on integrated or secreted MUC1 glycoforms from carcinoma cells and human milk. BW835-reactive glycopeptides on MUC1 have been identified. The epitope of BW835 was localized to threonine within the VTSA-peptide motif by site-specific enzymatic beta-galactosylation of the synthetic tandem repeat peptide TAP25-GalNAc1 TAPPAHGVT(-O-alpha GalNAc)SAPDTRPAPGSTAPPA (Hanisch FG, Stadie T, Boßlet K. Cancer Res. 1995;55:4036-40).

EP0528767A1 discloses the use of a human/mouse chimeric and humanized monoclonal antibodies recognizing the Lewis y antigen, represented by the difucosyl Lewis blood group antigens Y-6 and B-7-2. Such antibodies are, for instance, antibodies containing the variable region of the murine antibody BR55-2, e.g. the humanised BR55-2, which is called IGN311 or VL311.

These antibodies are specifically defined by their CDR binding site with complementarity defining regions of the light chain sequence
CDR1: RSSQSIVHSNGNTYLE (SEQ ID No. 1)
CDR2: KVSNRFS (SEQ ID No. 2)
CDR3: FQGSHVPFT (SEQ ID No. 3), and of the heavy chain sequence
CDR1: DYYMY (SEQ ID No. 4)
CDR2: YISNGGGSSHYVDSVKG (SEQ ID No. 5)
CDR3: GMDYGAWFAY (SEQ ID No. 6).

The complement dependent cytotoxicity (CDC) activity of the humanized anti Lewis-y antibody IGN311 was described by Nechansky et at (J Pharm Biomed Anal. 2009 May 1;49(4): 1014-20. Epub 2009 Feb 4) demonstrating the cytotoxic effect as measured in a FACS-based CDC assay.

WO04/016285A1 describes a kit for the combined use for the treatment of cancer patients, which set comprises an antibody directed against the aberrant glycosylation, such as IGN311, and an antibody directed against the cellular surface protein, for the immunotherapeutic and the diagnostic application.

A mutated Lewis y specific antibody is disclosed in WO06/005367A1. By engineering the Fc region of said antibody it carries a bi-sected hybrid type N-glycosylation pattern resulting in increased ADCC and decreased CDC activities.

An *in vivo* glyco-engineered anti-Lewis y antibody with improved lytic potential produced by a glyco-optimized strain of the moss *Physcomitrella patens* is described by Schuster et al (Biotechnology Journal, Volume 2 Issue 6, Pages 700 - 708, Special Issue: Biopharmaceutical Technologies, Published Online: 12 Apr 2007). The glycoengineered IGN311 antibody transiently expressed and secreted by such genetically modified moss protoplasts assembled correctly, showed an unaltered antigen-binding affinity and revealed an enhanced ADCC.

Other immunoglobulins that have been widely used for treating patients are targeting a receptor of the erbB class. Among those receptors are EGFR (Her1), Her2, Her2neu, Her3 and Her4.

Herceptin (trastuzumab, humAb4D5) is a product based on a monoclonal antibody for use in breast cancer therapy. Herceptin antibody is specific for the 4D5 epitope of the HER2 extracellular domain of her2neu (also called c-erbB-2 or MAC117).

"HER2 extracellular domain" or "HER2 ECD" refers to a domain of HER2 that is outside of a cell, either anchored to a cell membrane, or in circulation, including fragments thereof. The extracellular domain of HER2 may comprise four domains: "Domain I" (amino acid residues from about 1-195, "Domain II" (amino acid residues from about 196-319), "Domain III" (amino acid residues from about 320-488), and "Domain IV" (amino acid residues from about 489- 630) (residue numbering without signal peptide).

The "epitope 4D5" is the region in the extracellular domain of HER2 to which the antibody 4D5 (ATCC CRL 10463) and trastuzumab bind. This epitope is close to the transmembrane domain of HER2, and within Domain IV of HER2. The 4D5 epitope of HER2 encompasses any one or more residues in the region from about residue 529 to about residue 625, inclusive of the HER2 ECD, residue numbering including signal peptide.

The EGFR is a large (1,186 residues), monomeric glycoprotein with a single transmembrane region and a cytoplasmic tyrosine kinase domain flanked by noncatalytic regulatory regions. Sequence analyses have shown that the ectodomain (residues 1-621) contains four sub-domains, here termed L1, CR1, L2 and CR2, where L and CR are acronyms for large and Cys-rich respectively. The L1 and L2 domains have also been referred to as domains I and III, respectively. The CR domains have been previously referred to as domains II and IV, or as S1.1-S1.3 and S2.1-S2.3 where S is an abbreviation for small.

MAbs to the external domain of the EGFR have been developed that disrupt ligand binding to the receptor and subsequent signal transduction. Three EGFRspecific blocking antibodies have been characterized in greater detail in vitro and are presently used in clinical studies; these are mAbC225 (ERBITUX/cetuximab), mAb425 (EMD72000) and the human mAb ABX-EGF. C225 (Cetuximab/Erbitux) is FDA approved for metastatic colorectal cancer and mAb425 (EMD59000) whose humanized version (EMD72000) is currently in phase II clinical trials for various solid tumors expressing EGFr. C225 binds to distinct epitopes on the extracellular domain of EGFr. Independent binding of both antibodies to the wild type receptor and to the mutant receptor (EGFrVIII) which is prominently expressed in tumor cells, has been shown. Cetuximab interacts exclusively with domain III of the extracellular region of EGFR (sEGFR), particularly occluding the ligand binding region on this domain and sterically preventing the receptor from dimerization.

The spontaneously occurring mutant EGF receptor was first shown in glioblastoma. Known as EGFRvIII, this molecule represents a deletion of exons 2 through 7 in the extracellular domain of the EGF receptor. This removes 273 amino acids and creates a novel glycine at the fusion junction. The EGFRvIII (variously called de2-7 EGFR or deltaEGFR) has an in-frame deletion of the extracellular domain and is found in numerous types of human tumors.

WO97/20858A1 relates to anti-Her2 antibodies which induce apoptosis in Her2 expressing cells. Therefore the monoclonal antibodies (mAbs), which bind to Her2, are generated by immunizing mice with purified soluble Her2.

WO06/087637A2 relates to antibodies that recognise Her2/neu and exert an antiproliferative effect on Her2/neu expressing cells. This document describes an isolated antibody or a fragment, variant or derivative thereof, in particular the human Fab fragment, and the scFv fragment, capable of specifically binding to Her2neu.

Some prior art disclosures relate to antibody formats with a potential to inhibit tumor growth, in the absence of cytotoxic activities, such as ADCC.

Rovers et al (Cancer Immunol. Immunother. (2007) 56:303-317) describe anti-EGFR nanobodies with a potential to inhibit tumour cell growth through a process called acinosis.

WO06/036834A2 describes a biologically active peptide incorporated as an internal sequence into a loop region of an Fc domain; the specification concerns a molecule of which the internal peptide sequence may be added by insertion or replacement of amino acids in the previously existing Fc domain. An exemplary peptide is targeting p185HER2/neu.

Antibodies typically mediate Antibody-Dependent Cell-Mediated Cytotoxicity (ADCC), which is a mechanism of cell-mediated immunity whereby an effector cell (for instance a natural killer cell, NK cell) of the immune system actively lyses a target cell that has been bound by specific antibodies. An NK cell's Fc receptor recognizes the Fc portion of an antibody, such as IgG, which has bound to the surface of a target cell. The most common Fc receptor on the surface of an NK Cell is called CD16a or FcyRlll. Once the Fc receptor binds to the Fc region of IgG, the NK cell releases cytokines such as IFN-γ, and cytotoxic granules containing perforin and granzymes that enter the target cell and promote cell death.

Phagocytic effector cells may be activated through another route employing activation of complement. Antibodies that bind to surface antigens on microorganisms attract the first component of the complement cascade with their Fc region and initiate activation of the "classical" complement system. This results in the stimulation of phagocytic effector cells, which ultimately kill the target by complement dependent cytotoxicity (CDC).

NK cell dysfunction or deficiency has been shown to lead to the development of autoimmune diseases (such as diabetes or atherosclerosis) and cancers. Lymphocytopenia is a frequent, temporary result from many types of chemotherapy, such as with cytotoxic agents or immunosuppressive drugs. Some malignancies in the bone marrow, such as leukemia, also cause lymphocytopenia. Large doses of radiation, such as used for treating tumors, may cause lymphocytopenia.

It is the object of present invention to provide improved immunoglobulin products that are capably of tumor cell lysis independent of available effector cells.

The object is solved by the subject matter as claimed.

### Summary of the Invention

According to the invention there is provided a modular antibody having at least two specificities to crosslink a glycoepitope and a receptor of the erbB class on a tumor cell, which antibody is essentially free of effector function in the tumor cell based assay, which is preferably a multispecific tumor cell based assay that employs the binding of tumor cells through the at least two specificities. Specifically preferred is the bispecific tumor cell assay, where the modular antibody according to the invention binds to the tumor cell expressing the glycoepitope and the receptor.

Specifically the first specificity is directed to a glycoepitope of a blood group related antigen. Specifically preferred target antigens are selected from the group consisting of Lewis x-, Lewis b- and Lewis y-structures, Globo H-structures, KH1, Tn antigen, TF antigen and carbohydrate structures of Mucins, CD44 including its splice variants, and phospholipids, glycolipids and glycosphingolipids, such as Gg3, Gb3, GD3, GD2, Gb5, Gm1, Gm2, sialyltetraosylceramide.

Preferably a second specificity is directed to an erbB receptor tyrosine kinase selected from the group consisting of EGFR, HER2, HER3 and HER4, preferably a human receptor. Specifically the relevant epitopes of the receptor are selected from extracellular or external domains of said receptor.

It is preferred that the modular antibody according to the invention contains a binding site having a randomized antibody sequence. Thereby the binding site may be randomly generated and a binder having suitable binding characteristics may be easily selected from a repertoire of variants. Accordingly the binding site may be produced through mutagenesis of a nucleotide or amino acid sequence. The site of the randomized antibody sequence may be within the CDR region or the structural loop region, which is always understood to potentially include a terminal domain sequence that could be contributing to antigen binding.

It is further preferred that the modular antibody according to the invention contains a binding site within a structural loop region.

The preferred format of the modular antibody according to the invention is an oligomer of modular antibody domains. Specifically said oligomer comprises immunoglobulin domains selected from the group consisting of VH/VL, CH1/CL, CH2/CH2, CH3/CH3, Fc, Fab and scFv.

According to a preferred embodiment the antibody is a bispecific full-length immunoglobulin or an antigen binding Fc molecule called Fcab.

The modular antibody according to the invention preferably is provided as an antibody that simultaneously binds to both, the glycoepitope and said receptor on a tumor cell. Simultaneous binding is preferably determined in a cell-based assay with two-dimensional differentiation, e.g. in a FACS system.

It is further preferred that the antibody binds to the glycoepitope and said receptor on a tumor cell by at least three binding sites.

The preferred modular antibody binds to said tumor cell with a Kd<10⁻⁸M. Specifically a high affinity Lewis y specific modular antibody is preferred which has at least one further specificity to crosslink a receptor of the erbB class of a tumor cell, which antibody binds to said tumor cell with a Kd<10⁻⁸M.

Specifically the modular antibody according to the invention has a binding site for specifically recognizing the Lewis y antigen, such as the difucosyl Lewis blood group antigens Y-6 and B-7-2, more preferably it has the Lewis y binding specificity of IGN311. In a preferred embodiment the modular antibody according to the invention has an antigen binding site comprising a significant part of the CDR sequences of BR55-2, or functional variants thereof or are consisting of said CDR sequences, including functional variants.

According to an alternative embodiment the modular antibody according to the invention has a binding site for specifically recognizing a glycopeptide of MUC1, such as the BW835 antigen. BW835 defines a carbohydrate epitope on integrated or secreted MUC1 glycoforms from carcinoma cells.

According to a specific aspect the modular antibody according to the invention is provided for the treatment of a patient suffering from a solid tumor, which tumor expresses a receptor of the erbB class and an aberrant glycosylation. Though the receptor expressed on tumor cells may bear the glycoepitope itself, the receptor is not necessarily glycosylated on specific tumor cells. It turned out that erbB on some of the cells which can be killed by the modular antibody according to the invention are either not glycosylated or that in addition to erbB other structures are also glycosylated.
Thus, the solid tumor disease is specifically treated wherein tumor cells overexpress at least one of the erbB family members and carry an aberrant (tumor-associated) glycoepitope on the same cell.

Specifically the modular antibody is used for the treatment of breast cancer, colorectal cancer, head and neck cancer or gastric cancer.

Preferably the modular antibody according to the invention is used for the treatment of immunocompromised patients, preferably in combination with chemotherapy or radiotherapy.

Thus, the modular antibody according to the invention is preferably provided for manufacturing a pharmaceutical preparation for the treatment of cancer, in particular breast cancer, colorectal cancer, head and neck cancer or gastric cancer.

It surprisingly turned out that the modular antibody according to the invention has apoptotic activity effecting cytolysis independent of NK cells. Thus, the modular antibody has improved anti-tumor killing activity, optionally in addition to its cytotoxic activity associated with ADCC and/ or CDC activity, and may therefore be provided specifically for the treatment of immunocompromised patients, such as those suffering from NK deficiency, e.g. transient or local leukocytopenia, e.g. resulting from medication. In accordance therewith, it is preferred to treat solid tumor patients in combination with chemotherapy or radiotherapy.

A preferred method of preparing a modular antibody according to the invention comprises the steps of
a. fusing or recombining the following components
   (i) a modular antibody with a specificity to bind at least a glycoepitope and
   (ii) a modular antibody with a specificity to bind at least a receptor of the erbB class,
   to obtain a modular antibody with at least both specificities, and
b. determining the cytolysis of said tumor cell in the absence of NK cells.

### Figures

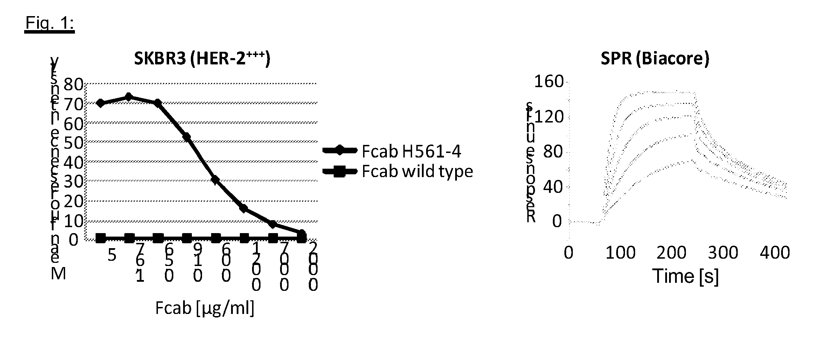

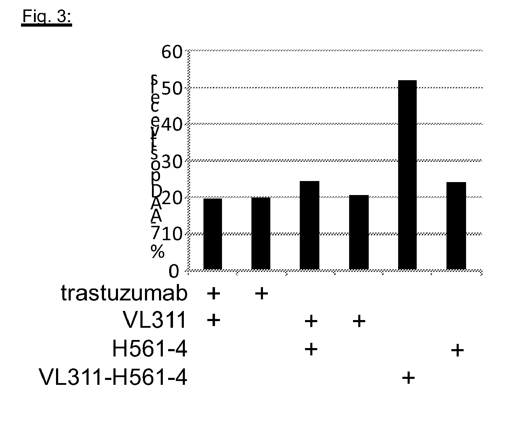

Figure 1:
   Results of binding affinity measurement of human Her-2 specific Fcab H561-4 determined by surface plasmon resonance (SPR) assays in a Biacore instrument. These experiments indicate that Fcab H561-4 has a binding affinity for recombinant HER-2 of 7.5nM (figure 1, right panel). Alternatively, binding of Fcab H561-4 to HER-2 expressed on human breast cancer cell line SKBR3 is determined. Fcab binding is enumerated by flow cytometry by plotting the mean fluorescence intensity against the Fcab concentrations (figure 1, left panel). These experiments indicate an apparent EC₅₀ binding for Fcab H561-4 of 2nM which is in good agreement with the SPR data.
Figure 2:
   In order to assess the synergistic effect of antibodies on tumor cell growth, human tumor cell lines expressing different levels of HER2, HER1, Lewis Y and the Thomsen-Friedenreich (TF) antigen are used (BT474, Calu-3 and MD-MBA468, obtained from LGC Standards). The data demonstrate that both parental antibodies have no effect on the growth of the three cell lines. By contrast, HER2 binding site containing mAb² are able to kill BT474 cells which express HER2, LeY and TF antigens while having no effect on MD-MBA468 cells which do not express HER2 but are positive for both glyco-epitopes. In contrast, both mAb² with the HER1 binding site are able to elicit cell death in MD-MBA468 cells which express high levels of HER1 and both Lewis Y and TF antigens. Low killing activity is seen with VL311-EAM151-5 in BT474 cells, presumably due to its high expression levels of Lewis Y. None of the mAb² is able to kill Calu-3 cells which do express both ErbB family members but are devoid of the two glyco-epitopes under study.
Figure 3:
   To determine, if Fcab H561-4 itself is responsible for the killing effect HCC1954 cells (HER2⁺⁺⁺, LeY⁺) are incubated with 18.5nM Fcab H561-4 alone. To further determine, if the way how HER2 and the Lewis Y antigen are engaged by antibodies plays a role for cell death induction, cells are treated with 6.25nM antibodies alone or in combinations as shown in figure 3. The data indicate that Fcab H561-4 alone had no effect on cell viability indicating that simultaneous binding of HER2 and Lewis Y is necessary for cell death induction. In addition, the mixture of VL311 and Fcab H561-4 or the mixture of VL311 and trastuzumab (trade name Herceptin, Genentech, a clinically approved HER-2 antibody) does not lead to any induction of cell death in contrast to mAb2 VL311-H561-4 which induces a robust killing response. This data demonstrate that the modality of simultaneous engagement of HER-2 and Lewis Y determines if HCC1954 cells will be killed or not. Co-crosslinking of HER2 and LewisY by a single molecular entity, such as the mAb², provides the necessary signal for inducing cell death.
Figure 4:
   To determine if the mechanism by which the mAb² proteins kill cells involves apoptosis, SKBR3 cells which express HER-2 and Lewis Y are incubated with increasing concentrations of parental VL311 mAb or mAb2 VL311-H561-4 for 24 hours at 37°C. Results of tests for the presence of Annexin V positivity using the FITC Annexin V Apoptosis Detection Kit I (Beckton Dickinson) and the "TUNEL" (dUTP nick end labeling) assay demonstrate that only mAb² VL311-H561-4, but not the parental antibody VL311 induces the appearance of Annexin V and dUTP positive cells indicative of early and later stages of apoptosis. Therefore, incubation of tumor cells with mAb² VL311-H561-4 kills cells by an apoptotic mechanism.
Figure 5:
   Amino acid sequences of
   BW835 VH (SEQ ID No. 7),
   BW835 VL (SEQ ID No. 8),
   VL311 VH (SEQ ID No. 9),
   VL311 VL (SEQ ID No. 10) and
   Fcab H561-4 (SEQ ID No. 11).

### Detailed Description of the Invention

Specific terms as used throughout the specification have the following meaning.

The term "immunoglobulin" as used according to the present invention is defined as polypeptides or proteins that may exhibit mono- or bi- or multi-specific, or mono-, bi- or multivalent binding properties, preferably at least two, more preferred at least three specific binding sites for epitopes of e.g. antigens, effector molecules or proteins either of pathogen origin or of human structure, like self-antigens including cell-associated or serum proteins. The term immunoglobulin as used according to the invention also includes functional fragments of an antibody, such as Fc, Fab, scFv, single chain dimers of CH1/CL domains, Fv, dimers like VH/VL, CH1/CL, CH2/CH2, CH3/CH3, or other derivatives or combinations of the immunoglobulins, like single chains of pairs of immunoglobulin domains. The definition further includes domains of the heavy and light chains of the variable region (such as dAb, Fd, VI, Vk, Vh, VHH) and the constant region or individual domains of an intact antibody such as CH1, CH2, CH3, CH4, Cl and Ck, as well as mini-domains consisting of at least two beta-strands of an immunoglobulin domain connected by a structural loop.

"Modular antibodies" as used according to the invention are defined as antigen-binding molecules, like human antibodies, composed of at least one polypeptide module or protein domain, preferably in the natural form. The term "modular antibodies" includes antigen-binding molecules that are either immunoglobulins, immunoglobulin-like proteins, or other proteins exhibiting modular formats and antigen-binding properties similar to immunoglobulins or antibodies, which can be used as antigen-binding scaffolds, preferably based on human proteins.

The term "immunoglobulin-like molecule" as used according to the invention refers to any antigen-binding protein, in particular to a human protein, which has a domain structure that can be built in a modular way. Immunoglobulin-like molecules as preferably used for the present invention are T-cell receptors (TCR) or soluble parts thereof, fibronectin, transferrin, CTLA-4, single-chain antigen receptors, e.g. those related to T-cell receptors and antibodies, antibody mimetics, adnectins, anticalins, phylomers, repeat proteins such as ankyrin repeats, avimers, Versabodies ™, scorpio toxin based molecules, and other non-antibody protein scaffolds with antigen binding properties.

Ankyrin repeat (AR), armadillo repeat (ARM), leucine-rich repeat (LRR) and tetratricopeptide repeat (TPR) proteins are the most prominent members of the protein class of repeat proteins. Repeat proteins are composed of homologous structural units (repeats) that stack to form elongated domains. The binding interaction is usually mediated by several adjacent repeats, leading to large target interaction surfaces.

Avimers contain A-domains as strings of multiple domains in several cellsurface receptors. Domains of this family bind naturally over 100 different known targets, including small molecules, proteins and viruses. Truncation analysis has shown that a target is typically contacted by multiple A-domains with each domain binding independently to a unique epitope. The avidity generated by combining multiple binding domains is a powerful approach to increase affinity and specificity, which these receptors have exploited during evolution.

Anticalins are engineered human proteins derived from the lipocalin scaffold with prescribed binding properties typical for humanized antibodies. Lipocalins comprise 160-180 amino acids and form conical beta-barrel proteins with a ligand-binding pocket surrounded by four loops. Small hydrophobic compounds are the natural ligands of lipocalins, and different lipocalin variants with new compound specificities, also termed 'anticalins', could be isolated after randomizing residues in this binding pocket.

Phylomers are peptides derived from biodiverse natural protein fragments.

It is understood that the term "modular antibody", "immunoglobulin", "immunoglobulin-like proteins" includes a derivative thereof as well. A derivative is any combination of one or more modular antibodies of the invention and or a fusion protein in which any domain or minidomain of the modular antibody of the invention may be fused at any position of one or more other proteins (such as other modular antibodies, immunoglobulins, ligands, scaffold proteins, enzymes, toxins and the like). A derivative of the modular antibody of the invention may also be obtained by association or binding to other substances by various chemical techniques such as covalent coupling, electrostatic interaction, di-sulphide bonding etc. The other substances bound to the immunoglobulins may be lipids, carbohydrates, nucleic acids, organic and inorganic molecules or any combination thereof (e.g. PEG, prodrugs or drugs). A derivative would also comprise an antibody with the same amino acid sequence but made completely or partly from non-natural or chemically modified amino acids. The term derivative also includes fragments and variants, which serve as functional equivalents. The preferred derivatives still are functional with regard to both, the Lewis y binding and the receptor binding on the target cell.

A "structural loop" or "non-CDR-loop" according to the present invention is to be understood in the following manner: modular antibodies, immunoglobulins or immunoglobulin-like substances are made of domains with a so called immunoglobulin fold. In essence, antiparallel beta sheets are connected by loops to form a compressed antiparallel beta barrel. In the variable region, some of the loops of the domains contribute essentially to the specificity of the antibody, i.e. the binding to an antigen by the natural binding site of an antibody. These loops are called CDR-loops. The CDR loops are located within the CDR loop region, which may in some cases also include part of the variable framework region (called "VFR"), which is adjacent to the CDR loops. It is known that some loops of the VFR may contribute to the antigen binding pocket of an antibody, which generally is mainly determined by the CDR loops. Thus, those VFR loops are considered as part of the CDR loop region, and would not be appropriately used for engineering new antigen binding sites. Loops aside from the antigen-binding pocket or CDR loop region are usually called structural loops or non-CDR-loops. Contrary to the VFR within the CDR loop region or located proximal to the CDR loops, other loops of the VFR of variable domains would be considered structural loops and particularly suitable for use according to the invention. Those are preferably the structural loops of the VFR located opposite to the CDR loop region, or at the C-terminal side of a variable immunoglobulin domain. Constant domains have structural loops within a structural loop region, e.g. either at the C-terminal side of an antibody domain or at an N-terminal side, even within a side chain of an antibody domain. Constant domains are also called part of the framework region. C-terminal amino acid sequences may also contribute to the non-CDR antigen binding, thus, are considered part of a structural loop region, which may be engineered to create a new antigen-binding site.

The term "antigen" or "target" as used according to the present invention shall in particular include all antigens and target molecules capable of being recognised by a binding site of a modular antibody. Specifically preferred antigens as targeted by the molecule according to the invention are those antigens or molecules, which have already been proven to be or are capable of being immunologically or therapeutically relevant, especially those, for which a clinical efficacy has been tested. The term "target" or "antigen" as used herein shall in particular comprise molecules selected from the group consisting of tumor associated antigens, which are self antigens, such as cell surface receptors or aberrant glycosylation patterns.

The term "glycoepitope" or "glycosylated epitope" as used herein shall refer to epitopes formed by carbohydrate chains on polypeptide and/or cell wall structures. Glycoepitopes were found to be involved in diverse functions as cell to cell recognition and communication in neuronal tissues and immune systems, pathogen recognition, sperm-egg recognition and fertilization, regulating hormonal half-lives in the blood, directing embryonic development and differentiation, and directing distribution of various cells and proteins throughout the body.

Specific glycoproteins and glycolipids have glycoepitopes which determine blood types. Blood group antigens (BGA)-related glycodeterminants are specific glycoepitopes expressed on the cell surface at definite stages of cell differentiation during embryogenesis, organogenesis, tissue repair, regeneration, remodeling and maturation when 'sorting-out' behaviour of one homotypic cell population from heterotypic assemblage of cells occurs. In this event the BGA-related glycoepitopes, if being expressed on the cell surface, play a role of key structural determinants in cell-cell recognition, association and aggregation. In cancer it has been considered as a key mechanism of phenotypic divergence of tumor cells, immunoselection, tumor progression and metastasis. There are three types of blood-group antigens: O, A, and B. They differ only slightly in the composition of carbohydrates.

The modular antibody according to the invention preferably binds to glycoepitope targets of aberrant carbohydrate structures on epithelial cancer cells. Among them are blood group antigen related glycoepitopes, such as Lewis x-, Lewis band Lewis y-structures, including sialylated Lewis x-structures. Other preferred carbohydrate targets are Globo H-structures, KH1, Tn antigen, TF antigen, such as the Thomsen-Friedenreich (TF)-disaccharide (Galβ1-3GalNAc-), β-galactoside sequences of several cell surface structures (e.g. Galβ1-4GlcNAc), the alpha-1,3-galactosyl epitope (Elektrophoresis (1999), 20:362; Curr. Pharmaceutical Design (2000), 6:485, Neoplasma (1996), 43:285), carbohydrate structures of Mucins, including MUC1 glycoforms, carbohydrate structures on CD44 including all splice variants thereof, and carbohydrates found on glycolipids and glycosphingolipids, such as Gg3, Gb3, GD3, GD2, Gb5, Gm1, Gm2, sialyltetraosylceramide.

Cell surface antigens are typically structures on the surface of a cell capable of being recognised by an antibody. Preferred cell surface antigens are those antigens, which have already been proven to be or which are capable of being immunologically or therapeutically relevant, especially those, for which a preclinical or clinical efficacy has been tested. Those cell surface molecules are specifically relevant for the purpose of the present invention, which mediate cell killing activity. Upon binding of the modular antibody according to the invention to the glycoepitope motif and at least one of the epitopes of a receptor, a potent means for attacking human cells may be provided.

The antigen is either recognized as a whole target molecule or as a fragment of such molecule, especially substructures, e.g. a polypeptide or carbohydrate structure of targets, generally referred to as "epitopes", e.g. B-cell epitopes, T-cell epitope), which are immunologically relevant, i.e. are also recognisable by natural or monoclonal antibodies. The term "epitope" as used herein according to the present invention shall in particular refer to a molecular structure which may completely make up a specific binding partner or be part of a specific binding partner to a binding site of modular antibody of the present invention. The term epitope may also refer to haptens. Chemically, an epitope may either be composed of a carbohydrate, a peptide, a fatty acid, an organic, biochemical or inorganic substance or derivatives thereof and any combinations thereof. If an epitope is a polypeptide, it will usually include at least 3 amino acids, preferably 8 to 50 amino acids, and more preferably between about 10-20 amino acids in the peptide. There is no critical upper limit to the length of the peptide, which could comprise nearly the full length of a polypeptide sequence of a protein. Epitopes can be either linear or conformational epitopes. A linear epitope is comprised of a single segment of a primary sequence of a polypeptide or carbohydrate chain. Linear epitopes can be contiguous or overlapping. Conformational epitopes are comprised of amino acids or carbohydrates brought together by folding of the polypeptide to form a tertiary structure and the amino acids are not necessarily adjacent to one another in the linear sequence. Specifically, epitopes are at least part of diagnostically relevant molecules, i.e. the absence or presence of an epitope in a sample is qualitatively or quantitatively correlated to either a disease or to the health status of a patient or to a process status in manufacturing or to environmental and food status. Epitopes may also be at least part of therapeutically relevant molecules, i.e. molecules which can be targeted by the specific binding domain which changes the course of the disease.

As used herein, the term "specificity" or "specific binding" refers to a binding reaction which is determinative of the cognate ligand of interest in a heterogeneous population of molecules. Thus, under designated conditions (e.g. immunoassay conditions), the modular antibody binds to its particular target and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10 fold different, preferably the difference is at least 100 fold, and more preferred a least 1000 fold.

The term "cytotoxic" or "cytotoxic activity" as used for the purpose of the invention shall refer to any specific molecule directed against cellular antigens that, when bound to the antigen, activates programmed cell death and triggers apoptosis. Besides the apoptotic activity the modular antibody according to the invention may as well mediate ADCC or CDC, which is of particular importance when the target cells are heterogeneous and would express the glycoepitope and the receptor antigens to a different extent or even express only one of the relevant targets on the cell surface. Thus, when apoptose is less potent due to a differential expression of the relevant antigens on the target cell, the preferred modular antibody according to the invention may still be effective by its activity on effector cells resulting in activation of cytotoxic T-cells or cells which mediate antibody-dependent cell cytotoxicity (ADCC), complement dependent cytotoxicity (CDC) and/or cellular phagocytosis (ADCP). Modular antibodies according to the invention thus kill antibody-coated target cells by inducing programmed cell death and/or by binding to Fc receptors of effector cells.

Antibody-Dependent Cell-Mediated Cytotoxicity (ADCC) is a mechanism of cell-mediated immunity whereby an effector cell of the immune system actively lyses a target cell that has been bound by specific antibodies. It is one of the mechanisms through which antibodies, as part of the humoral immune response, can act to limit and contain infection. Classical ADCC is mediated by NK cells; monocytes and eosinophils can also mediate ADCC. ADCC is part of the adaptive immune response due to its dependence on a prior antibody response.

The term "foreign" in the context of amino acids shall mean the newly introduced amino acids being naturally occurring, but foreign to the site of modification, or substitutes of naturally occurring amino acids. "Foreign" with reference to an antigen binding sites means that the antigen binding site is not naturally formed by the specific binding region of the agent, and a foreign binding partner, but not the natural binding partner of the agent, is bound by the newly engineered binding site.

The term "variable binding region" sometimes called "CDR region" as used herein refers to molecules with varying structures capable of binding interactions with antigens. Those molecules can be used as such or integrated within a larger protein, thus forming a specific region of such protein with binding function. The varying structures can be derived from natural repertoires of binding proteins such as immunoglobulins or phylomers or synthetic diversity, including repeat-proteins, avimers and anticalins. The varying structures can as well be produced by randomization techniques, in particular those described herein. These include mutagenized CDR or non-CDR regions, loop regions of immunoglobulin variable domains or constant domains.

Modified binding agents with different modifications at specific sites are referred to as "variants". Variants of a scaffold are preferably grouped to form libraries of binding agents, which can be used for selecting members of the library with predetermined functions. In accordance therewith, an antibody sequence is preferably randomized, e.g. through mutagenesis methods. According to a preferred embodiment a loop region of a binding agent, such as the parent antibody sequence comprising positions within one or more loops or at a terminal site, potentially contributing to a binding site, is preferably mutated or modified to produce libraries, preferably by random, semi-random or, in particular, by site-directed random mutagenesis methods, thus, resulting in a randomized sequence, in particular to delete, exchange or introduce randomly generated inserts into loops or a loop region, preferably into the CDR loop region or structural loop region, which may include terminal sequences, that are located at one of the termini of an antibody domain or substructure.

Alternatively preferred is the use of combinatorial approaches. Any of the known mutagenesis methods may be employed, among them cassette mutagenesis. These methods may be used to make amino acid modifications at desired positions of the immunoglobulin of the present invention. In some cases positions are chosen randomly, e.g. with either any of the possible amino acids or a selection of preferred amino acids to randomize loop sequences, or amino acid changes are made using simplistic rules. For example all residues may be mutated preferably to specific amino acids, such as alanine, referred to as amino acid or alanine scanning. Such methods may be coupled with more sophisticated engineering approaches that employ selection methods to screen higher levels of sequence diversity.

The term "functionally equivalent variant" or "functionally active variant" of a modular antibody as used herein means a sequence resulting from modification of this sequence by insertion, deletion or substitution of one or more amino acids or nucleotides within the sequence or at either or both of the distal ends of the sequence, and which modification does not affect (in particular impair) the activity of this sequence. In the case of a binding site having specificity to a selected target antigen, the functionally active variant of a modular antibody according to the invention would still have the predetermined binding specificity, though this could be changed, e.g. to change the fine specificity to a specific epitope, the affinity, the avidity, the Kon or Koff rate, etc. In a preferred embodiment the functionally active variant a) is a biologically active fragment of the modular antibody, the functionally active fragment comprising at least 50% of the sequence of the modular antibody, preferably at least 70%, more preferably at least 80%, still more preferably at least 90%, even more preferably at least 95% and most preferably at least 97%, 98% or 99%; b) is derived from the modular antibody by at least one amino acid substitution, addition and/or deletion, wherein the functionally active variant has a sequence identity to the modular antibody or its relevant antibody domain or the antigen binding site of at least 50%, preferably at least 60%, more preferably at least 70%, more preferably at least 80%, still more preferably at least 90%, even more preferably at least 95% and most preferably at least 97%, 98% or 99%; and/or c) consists of the modular antibody or a functionally active variant thereof and additionally at least one amino acid or nucleotide heterologous to the polypeptide or the nucleotide sequence, preferably wherein the functionally active variant is derived from or identical to any of the naturally occurring variants of any of the sequences of SEQ ID No. 1, 2, 3 and/or 4. and variants derived from the CDR sequences of the BR55-2 or IGN311 antibody.

Functionally active variants may be obtained by changing the sequence as defined above and are characterized by having a biological activity similar to that displayed by the respective sequence, including the ability to bind the glycosylated epitope and receptor, respectively.

The functionally active variant may be obtained by sequence alterations in the polypeptide or the nucleotide sequence, wherein the sequence alterations retains a function of the unaltered polypeptide or the nucleotide sequence, when used in combination of the invention. Such sequence alterations can include, but are not limited to, (conservative) substitutions, additions, deletions, mutations and insertions.

The variant of the polypeptide or the nucleotide sequence is functionally active in the context of the present invention, if the activity of the composition of the invention including the variant (but not the original) amounts to at least 10%, preferably at least 25%, more preferably at least 50%, even more preferably at least 70%, still more preferably at least 80%, especially at least 90%, particularly at least 95%, most preferably at least 99% of the activity of the apoptotic modular antibody of the invention including the polypeptide or the nucleotide sequence without sequence alteration (i.e. the original polypeptide or the nucleotide sequence).

In one preferred embodiment of the invention, the functionally active variant of the modular antibody of the invention is essentially identical to the polypeptide or the nucleotide sequence described above, but differs from the polypeptide or the nucleotide sequence, respectively, in that it is derived from a homologous sequence of a different species. These are referred to as naturally occurring variants.

The term "functionally active variant" also includes naturally occurring allelic variants, as well as mutants or any other non-naturally occurring variants. As is known in the art, an allelic variant is an alternate form of a (poly)peptide that is characterized as having a substitution, deletion, or addition of one or more amino acids that does essentially not alter the biological function of the polypeptide.

In a preferred embodiment, the functionally active variant derived from the modular antibody as defined above by amino acid exchanges, deletions or insertions may also conserve, or more preferably improve, the activity.

Conservative substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.

In another embodiment of the invention the polypeptide or the nucleotide sequence as defined above may be modified by a variety of chemical techniques to produce derivatives having essentially the same activity (as defined above for fragments and variants) as the modified modular antibody, and optionally having other desirable properties.

As used herein, a "homologue" or "functional homologue" of a polypeptide shall mean that polypeptides have the same or conserved residues at a corresponding position in their primary, secondary or tertiary structure. The term also extends to two or more nucleotide sequences encoding homologous polypeptides. In particular, homologous compounds usually have at least about 50% amino acid sequence identity with regard to a full-length native sequence or any fragment thereof. Preferably, a homologous compound will have at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90% amino acid sequence identity, more preferably at least about 95% amino acid sequence identity to a native compound, or any other specifically defined fragment of a full-length compound. When the function as an apoptotic modular antibody is proven with such a homologue, the homologue is called "functional homologue".

The term "homologous nucleotide sequences" as used herein refers to nucleotide sequences which are related but not identical in their nucleotide sequence with the contemplated nucleotide sequence, and perform essentially the same function. These are also meant to encompass variations in its nucleotide composition including variations due to the degeneracy of the genetic code, whereby the nucleotide sequence performs essentially the same function.

"Percent (%) amino acid sequence identity" with respect to the polypeptide sequences identified herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

The modular antibody according to the invention surprisingly exerts a direct cytotoxicity, which is independent of NK cells. Through binding to at least both specificities, the glycoepitope and the receptor of the erbB class (herein called "receptor"), direct cell lysis was observed, which has proven to be synergistic when binding to both targets. This was shown with tumor cells expressing both targets. A mixture of antibodies, each directed to a single target was not effective. Only the cross-linking of the targets through the binding sites of the modular antibody was effectively killing the cell in an apoptosis assay.

A cytotoxic compound is effective in an apoptosis assay by activating a genetic program of controlled cell death. Apoptosis is characterized by well defined cytological and molecular events including a change in the refractive index of the cell, cytoplasmic shrinkage, nuclear condensation and cleavage of DNA into regularly sized fragments. Cells that are undergoing apoptosis shut down metabolism, lose membrane integrity and form membrane blebs.

The apoptotic activity is preferably measured using standard methods of determinating dying and/or dead cells. In order to measure apoptosis, cytotoxicity assays can be employed. These assays are can be radioactive and non-radioactive assays that measure increases in plasma membrane permeability, since dying cells become leaky or colorimetric assays that measure reduction in the metabolic activity of mitochondria; mitochondria in dead cells cannot metabolize dyes, while mitochondria in live cells can.

One can also measure early indicators for apoptosis such as alterations in membrane asymmetry resulting in occurrence of phosphatidylserine on the outside of the cell surface (Annexin V based assays). Alternatively, later stages of apoptosis, such as activation of caspases can be measured in populations of cells or in individual cells. In addition, measurement of release of cytochrome C and AIF into cytoplasm by mitochondria or fragmentation of chromosomal DNA can be determined.

Terminal deoxynucleotidyl transferase dUTP nick end labeling (TUNEL) is a common method for detecting DNA fragmentation that results from apoptotic signaling cascades. The assay relies on the presence of nicks in the DNA which can be identified by terminal deoxynucleotidyl transferase, an enzyme that will catalyze the addition of bromolated dUTPs that are secondarily detected with a specific labelled antibody

The preferred apoptotic activity of the modular antibody according to the invention amounts to at least 20% of cytolysis, preferably at least 30%, more preferred at least 40%, even more preferred at least 50%, as measured in a respective *ex vivo* cell killing assay.

Though there was a long term need for highly effective cancer immunotherapy, prior art methods mainly relied on ADCC or CDC to kill cancer cells. Any apoptotic activity was considered too weak for anti-tumor therapy. A systemic or local deficiency of lymphocytes or NK cells, however, significantly hampers the chances of a successful therapy. Chemotherapy or radiotherapy is commonly used in treatment of solid tumor disease, which evidently rendered the patients immunocompromised. On the other hand, antibody therapy may lead to local NK cell deficiency at the tumor site because of effector cell consummation due to the antibody's effector function mediating ADCC and/ or CDC activity. The subject matter of the present invention avoids the disadvantages of the prior art immunotherapies and provides for an effective immunotherapy in solid tumor disease.

According to a specific embodiment of the present invention the modular antibody is an immunoglobulin of human or murine origin, or a humanized or chimeric immunoglobulin, which may be employed for various purposes, in particular in pharmaceutical compositions.

The human immunoglobulin, is preferably selected or derived from the group consisting of IgA1, IgA2, IgD, IgE, IgG1, IgG2, IgG3, IgG4 and IgM. The murine immunoglobulin binding agent is preferably selected or derived from the group consisting of IgA, IgD, IgE, IgG1, IgG2A, IgG2B, IgG2C, IgG3 and IgM.

A modular antibody according to the invention may comprise a heavy and/or light chain, at least one variable and/or constant domain, or a part thereof including a minidomain.

A constant domain is an immunoglobulin fold unit of the constant part of an immunoglobulin molecule, also referred to as a domain of the constant region (e.g. CH1, CH2, CH3, CH4, Ck, Cl).

A variable domain is an immunoglobulin fold unit of the variable part of an immunoglobulin, also referred to as a domain of the variable region (e.g. Vh, Vk, Vl, Vd)

The modular antibody according to the invention preferably is derived from the immunoglobulin structure, such as a full length immunoglobulin. The modular antibody according to the present invention may comprise one or more domains (e.g. at least two, three, four, five, six, ten domains). The preferred format is an oligomer, composed of modular antibody domains, preferably 2 to 12 domains, more preferred at least 4 domains, which oligomer preferably comprises a heterodimer, such as Fab, or a homodimer, such as Fc.

It is feasible to provide the preferred modular antibody of the invention as a single domain antibody. However, antibody domains tend to dimerize upon expression, either as a homodimer, like an Fc, or a heterodimer, like an Fab. The dimeric structure is thus considered advantageous to provide a stable molecule. The preferred dimers of immunoglobulin domains are selected from the group consisting of single domain dimers, like VH/VL, CH1/CL (kappa or lambda), CH2/CH2 and CH3/CH3. Dimers or oligomers of modular antibody domains can also be provided as single chain or two chain molecules, in particular those linking the C-terminus of one domain to the N-terminus of another.

If more than one domain is present in the modular antibody these domains may be of the same type or of varying types (e.g. CH1-CH1-CH2, CH3-CH3, (CH2)₂-(CH3)₂, with or without the hinge region). Of course also the order of the single domains may be of any kind (e.g. CH1-CH3-CH2, CH4-CH1-CH3-CH2).

The invention preferably refers to part of antibodies, such as parts of IgG, IgA, Igm, IgD, IgE and the like. The modular antibodies of the invention may also be a functional antibody fragment such as Fab, Fab₂, scFv, Fv, Fc, Fcab^{™}, an antigen-binding Fc, or parts thereof, or other derivatives or combinations of the immunoglobulins such as minibodies, domains of the heavy and light chains of the variable region (such as dAb, Fd, VL, including Vlambda and Vkappa, VH, VHH) as well as mini-domains consisting of two beta-strands of an immunoglobulin domain connected by at least two structural loops, as isolated domains or in the context of naturally associated molecules. A particular embodiment of the present invention refers to the Fc fragment of an antibody molecule, either as antigen-binding Fc fragment (Fcab^{™}) through modifications of the amino acid sequence or as conjugates or fusions to receptors, peptides or other antigen-binding modules, such as scFv.

An exemplary modular antibody according to the invention comprises a constant domain selected from the group consisting of CH1, CH2, CH3, CH4, Igk-C, Igl-C, combinations, derivatives or a part thereof including a mini-domain, with at least one structural loop region, and is characterised in that said at least one loop region comprises at least one amino acid modification forming at least one modified loop region, wherein said at least one modified loop region binds specifically to at least one epitope of an antigen.

Another modular antibody according to the invention can comprises a variable domain of a heavy or light chain, combinations, derivatives or a part thereof including a minidomain, with at least one variable and/or structural loop region, and is characterised in that said at least one loop region comprises at least one amino acid modification forming at least one modified loop region, wherein said at least one modified loop region binds specifically to at least one epitope of an antigen.

The modular antibodies can be used as isolated polypeptides or as combination molecules, e.g. through recombination, fusion or conjugation techniques, with other peptides or polypeptides. The peptides are preferably homologous to immunoglobulin domain sequences, and are preferably at least 5 amino acids long, more preferably at least 10 or even at least 50 or 100 amino acids long, and constitute at least partially the loop region of the immunoglobulin domain. The preferred binding characteristics relate to predefined epitope binding, affinity and avidity.

The modular antibody according to the invention is possibly further combined with one or more modified modular antibodies or with unmodified modular antibodies, or parts thereof, to obtain a combination modular antibody. Combinations are preferably obtained by recombination techniques, but also by binding through adsorption, electrostatic interactions or the like, or else through conjugation or chemical binding with or without a linker. The preferred linker sequence is either a natural linker sequence or functionally suitable artificial sequence. By such combination it is possible to link the structures responsible for the individual binding specificities, thereby providing for the crosslinking of the target structures on the cell surface.

In general, the modular antibody according to the invention may be used as a building block to molecularly combine other modular antibodies or biologically active substances or molecules. It is preferred to molecularly combine at least one antibody binding to the specific partner via the variable or non-variable sequences, like structural loops, with at least one other binding molecule which can be an antibody, antibody fragment, a soluble receptor, a ligand or another antibody domain, or a binding moiety thereof. Other combinations refer to proteinaceous molecules, nucleic acids, lipids, organic molecules and carbohydrates.

The engineered molecules according to the present invention will be useful as stand-alone molecules, as well as fusion proteins or derivatives, most typically fused before or after modification in such a way as to be part of larger structures, e.g. of complete antibody molecules, or parts thereof. Immunoglobulins or fusion proteins as produced according to the invention thus also comprise Fc fragments, Fab fragments, Fv fragments, single chain antibodies, in particular single-chain Fv fragments, bi- or multispecific scFv, diabodies, unibodies, multibodies, multivalent or multimers of immunoglobulin domains and others. It will be possible to use the engineered proteins to produce molecules which are monospecific, bispecific, trispecific, and may even carry more specificities. By the invention it is be possible to control and preselect the valency of binding at the same time according to the requirements of the planned use of such molecules.

According to the present invention, the modular antibody optionally exerts further binding regions to antigens, including the binding site binding specifically to a cell surface target and binding sites mediating effector function. Antigen binding sites to one or more antigens may be presented by the CDR-region or any other natural receptor binding structure, or be introduced into a structural loop region of an antibody domain, either of a variable or constant domain structure. The antigens as used for testing the binding properties of the binding sites may be naturally occurring molecules or chemically synthesized molecules or recombinant molecules, either in solution or in suspension, e.g. located on or in particles such as solid phases, on or in cells or on viral surfaces. It is preferred that the binding of an immunoglobulin to an antigen is determined when the antigen is still adhered or bound to molecules and structures in the natural context. Thereby it is possible to identify and obtain those modified immunoglobulins that are best suitable for the purpose of diagnostic or therapeutic use.

It is particularly preferred that the modular antibody according to the invention is capable of binding to said receptor through at least a structural loop region.

It is further preferred that the modular antibody according to the invention is capable of binding to said glycoepitope structure through at least a CDR region. The preferred modular antibody according to the invention has a CDR binding specificity of BR55-2 antibody or lGN31 antibody, including chimeric, humanized or human, which may be glycoengineered.

The modular antibody according to the invention may specifically bind to any kind of antigens, in particular to epitope structures derived from proteinaceous molecules, proteins, peptides, polypeptides, but also nucleic acids, glycans and carbohydrates. The preferred modular antibody according to the invention may comprise at least two loops or loop regions whereby each of the loops or loop regions may specifically bind to different molecules or epitopes.

Preferably a target antigen is selected from cell surface antigens, including receptors, in particular from the group consisting of erbB receptor tyrosine kinases (such as EGFR, HER2 including Her2neu, HER3 and HER4, in particular those epitopes of the extracellular domains of such receptors, e.g. the 4D5 epitope). In addition further antigens may be targeted, e.g. molecules of the TNF-receptor superfamily, such as Apo-1 receptor, TNFR1, TNFR2, nerve growth factor receptor NGFR, CD40, CD40-Ligand, OX40, TACI, BCMA, BAFF-receptor, T-cell surface molecules, T-cell receptors, T-cell antigen, Apo-3, DR4, DR5, DR6, decoy receptors ,such as DcR1, DcR2, CAR1, HVEM, GITR, ZTNFR-5, NTR-1, TNFL1, IGFR-1, c-Met, but not limited to these molecules, B-cell surface antigens, such as CD10, CD19, CD20, CD21, CD22, , DC-SIGN, antigens or markers of solid tumors or hematologic cancer cells, cells of lymphoma or leukaemia, other blood cells including blood platelets, but not limited to these molecules.

According to a further preferred embodiment a target antigen is selected from those antigens presented by cells, like epithelial cells or cells of solid tumors. Those target antigens expressed or overexpressed by cells are preferably targeted, which are selected from the group consisting of tumor associated antigens, in particular EpCAM, tumor-associated glycoprotein-72 (TAG-72), tumor-associated antigen CA 125, Prostate specific membrane antigen (PSMA), High molecular weight melanomaassociated antigen (HMW-MAA), tumor-associated antigen expressing Lewis y related carbohydrate, Carcinoembryonic antigen (CEA), CEACAM5, HMFG PEM, mucin MUC1, MUC18 and cytokeratin tumor-associated antigen, CD44 and its splice variants, bacterial antigens, viral antigens, allergens, allergy related molecules IgE, cKIT and Fc-epsilon-receptorl, IRp60, IL-5 receptor, CCR3, red blood cell receptor (CR1), human serum albumin, mouse serum albumin, rat serum albumin, Fc receptors, like neonatal Fc-gamma-receptor FcRn, Fc-gamma-receptors Fc-gamma RI, Fc-gamma-RII, Fc-gamma RIII, Fc-alpha-receptors, Fc-epsilon-receptors, fluorescein, lysozyme, toll-like receptor 9, erythropoietin, CD2, CD3, CD3E, CD4, CD11, CD11a, CD14, CD16, CD18, CD19, CD20, CD22, CD23, CD25, CD28, CD29, CD30, CD32, CD33 (p67 protein), CD38, CD40, CD40L, CD52, CD54, CD56, CD64, CD80, CD147, GD3, IL-1, IL-1R, IL-2, IL-2R, IL-4, IL-5, IL-6, IL-6R, IL-8, IL-12, IL-15, IL-17, IL-18, IL-23, LIF, OSM, interferon alpha, interferon beta, interferon gamma; TNF-alpha, TNFbeta2, TNFalpha, TNFalphabeta, TNF-R1, TNF-RII, FasL, CD27L, CD30L, 4-1 BBL, TRAIL, RANKL, TWEAK, APRIL, BAFF, LIGHT, VEG1, OX40L, TRAIL Receptor-1, A1 Adenosine Receptor, Lymphotoxin Beta Receptor, TACI, BAFF-R, EPO; LFA-3, ICAM-1, ICAM-3, integrin beta1, integrin beta2, integrin alpha4/beta7, integrin alpha2, integrin alpha3, integrin alpha4, integrin alpha5, integrin alpha6, integrin alphav, alphaVbeta3 integrin, FGFR-3, Keratinocyte Growth Factor, GM-CSF, M-CSF, RANKL, VLA-1, VLA-4, L-selectin, anti-ld, E-selectin, HLA, HLA-DR, CTLA-4, T cell receptor, B7-1, B7-2, VNRintegrin, TGFbeta1, TGFbeta2, eotaxin1, BLyS (B-lymphocyte Stimulator), complement C5, IgE, IgA, IgD, IgM, IgG, factor VII, CBL, NCA 90, EGFR (ErbB-1), Her2/neu (ErbB-2), Her3 (ErbB-3), Her4 (ErbB4), Tissue Factor, VEGF, VEGFR, endothelin receptor, VLA-4, carbohydrates such as blood group antigens and related carbohydrates, Galili-Glycosylation, Gastrin, Gastrin receptors, tumor associated carbohydrates, Hapten NP-cap or NIP-cap, T cell receptor alpha/beta, E-selectin, P-glycoprotein, MRP3, MRP5, glutathione-S-transferase pi (multi drug resistance proteins), alpha-granule membrane protein(GMP) 140, digoxin, placental alkaline phosphatase (PLAP) and testicular PLAP-like alkaline phosphatase, transferrin receptor, Heparanase I, human cardiac myosin, Glycoprotein IIb/IIIa (GPIIb/IIIa), human cytomegalovirus (HCMV) gH envelope glycoprotein, HIV gp120, HCMV, respiratory syncytial virus RSV F, RSVF Fgp, VNRintegrin, Hep B gp120, CMV, gpIIbIIIa, HIV IIIB gp120 V3 loop, respiratory syncytial virus (RSV) Fgp, Herpes simplex virus (HSV) gD glycoprotein, HSV gB glycoprotein, HCMV gB envelope glycoprotein, Clostridium perfringens toxin and fragments thereof.

In a preferred embodiment the modular antibody, besides having the glycoepitope specificity, is capable of binding to at least two relevant target epitopes, which are identical or differ from each other and are targeting the same or different type of tumor associated antigen, e.g. to EGFR and HER2, or HER2 and HER3 through its native, modified or newly formed binding site.

A modular antibody or immunoglobulin domains may be modified to change existing antigen binding sites or to add new antigen binding sites, which modifications are preferably effected in immunoglobulin domains or parts thereof that are either terminal sequences, preferably a C-terminal sequence, and/or part of a loop region, which contains a loop, either a CDR-loop or a non-CDR loop, structural loops being the preferred sites of modifications or mutagenesis. According to a specific embodiment the structural loop region also includes a terminal sequence, which contributes to antigen binding. In some cases it is preferable to use a defined modified structural loop or a structural loop region, or parts thereof, as isolated molecules for binding or combination purposes.

Specific modifications of the nucleic acid or amino acid sequences in a predetermined region, which result from random insertion or exchange or deletion of amino acids, either a selection of amino acids or the whole range of natural or synthetic amino acids, will result in a "randomized" sequence of a modular antibody according to the invention.

In a domain structure of a modular antibody it is preferred to modify or randomize the modular antibody within at least one loop region or terminal region, resulting in a substitution, deletion and/or insertion of one or more nucleotides or amino acids, preferably a point mutation, or even the exchange of whole loops, more preferred the change of at least 2, 3, 4, 5, 6, 7, 8, 9, 10, 11, 12, 13, 14 or 15, up to 30 amino acids. Thereby the modified sequence comprises amino acids not included in the conserved regions of the loops, the newly introduced amino acids being naturally occurring, but foreign to the site of modification, or substitutes of naturally occurring amino acids.

However, the maximum number of amino acids inserted into a loop region of a binding agent preferably may not exceed the number of 30, preferably 25, more preferably 20 amino acids at a maximum. The substitution and the insertion of the amino acids occurs preferably randomly or semi-randomly using all possible amino acids or a selection of preferred amino acids for randomization purposes, by methods known in the art and as disclosed in the present patent application.

The site of modification may be at a specific single loop or a loop region, in particular a structural loop or a structural loop region. A loop region usually is composed of at least two, preferably at least 3 or at least 4 loops that are adjacent to each other, and which may contribute to the binding of an antigen through forming an antigen binding site or antigen binding pocket. It is preferred that the one or more sites of modification are located within the area of 10 amino acids, more preferably within 20, 30, 40, 50, 60, 70, 80, 90 up to 100 amino acids, in particular within a structural region to form a surface or pocket where the antigen can sterically access the loop regions.

In this regard the preferred modifications are engineered in the loop regions of CH1, CH2, CH3 and CH4, in particular in the range of amino acids 7 to 21, amino acids 25 to 39, amino acids 41 to 81, amino acids 83 to 85, amino acids 89 to 103 and amino acids 106 to 117, or within the terminal sequences, preferably within 6 amino acids from the C- or N-terminus of the antibody domain.

In another preferred embodiment a modification in the structural loop region comprising amino acids 92 to 98 is combined with a modification in the structural loop region comprising amino acids 8 to 20.

The above identified amino acid regions of the respective immunoglobulins comprise loop regions to be modified. Preferably, a modification in the structural loop region comprising amino acids 92 to 98 is combined with a modification in one or more of the other structural loops.

In a preferred embodiment a modification in the structural loop region comprising amino acids 92 to 98 is combined with a modification in the structural loop region comprising amino acids 41 to 45.2.

Most preferably each of the structural loops comprising amino acids 92 to 98, amino acids 41 to 45.2 and amino acids 8 to 20 contain at least one amino acid modification.

In another preferred embodiment each of the structural loops comprising amino acids 92 to 98, amino acids 41 to 45.2, and amino acids 8 to 20 contain at least one amino acid modification.

According to another preferred embodiment the amino acid residues in the area of positions 15 to 17, 29 to 34, 41 to 45.2, 84 to 85, 92 to 100, and/or 108 to 115 of CH3 are modified.

The preferred modifications of Igk-C and Igl-C of human origin are engineered in the loop regions in the area of amino acids 8 to 20, amino acids 26 to 36, amino acids 41 to 82, amino acids 83 to 88, amino acids 92 to 100, amino acids 107 to 124 and amino acids 123 to 126, or within the terminal sequences, preferably within 6 amino acids from the C- or N-terminus of the antibody domain.

The preferred modifications of loop regions of Igk-C and Igl-C of murine origin are engineered at sites in the area of amino acids 8 to 20, amino acids 26 to 36, amino acids 43 to 79, amino acids 83 to 85, amino acids 90 to 101, amino acids 108 to 116 and amino acids 122 to 126.

Another preferred immunoglobulin preferably used as a therapeutic according to the invention consists of a variable domain of a heavy or light chain, or a part thereof including a minidomain, with at least one loop region, preferably a structural loop region, and is characterised in that said at least one loop region comprises at least one amino acid modification forming at least one modified loop region, wherein said at least one modified loop region forms a relevant binding site as described above.

According to a specific embodiment the immunoglobulin preferably used according to the invention may contain a modification within the variable domain, which is selected from the group of VH, Vkappa, VIambda, VHH and combinations thereof. More specifically, they comprise at least one modification within amino acids 7 to 22, amino acids 39 to 55, amino acids 66 to 79, amino acids 77 to 89 or amino acids 89 to 104, where the numbering of the amino acid position of the domains is that of the IMGT, or within the terminal sequences, preferably within 6 amino acids from the C- or N-terminus of the antibody domain.

In a specific embodiment, the immunoglobulin preferably used according to the invention is characterised in that the loop regions of VH or Vkappa or Vlambda of human origin comprise at least one modification within amino acids 7 to 22, amino acids 43 to 51, amino acids 67 to 77, amino acids 77 to 88, and amino acids 89 to 104, most preferably amino acid positions 12 to 17, amino acid positions 45 to 50, amino acid positions 68 to 77, amino acids 79 to 88, and amino acid positions 92 to 99, where the numbering of the amino acid position of the domains is that of the IMGT.

The structural loop regions of the variable domain of the immunoglobulin of human origin, as possible selected for modification purposes are preferably located in the area of amino acids 8 to 20, amino acids 44 to 50, amino acids 67 to 76, amino acids 78 to 87, and amino acids 89 to 101, or within the terminal sequences, preferably within 6 amino acids from the C- or N-terminus of the antibody domain.

According to a preferred embodiment the structural loop regions of the variable domain of the immunoglobulin of murine origin as possible selected for modification purposes are preferably located in the area of amino acids 6 to 20, amino acids 43 to 52, amino acids 67 to 79, amino acids 79 to 87, and amino acids 91 to 100, or within the terminal sequences, preferably within 6 amino acids from the C- or N-terminus of the antibody domain.

The immunoglobulin preferably used according to the invention may also be of camelid origin. Camel antibodies comprise only one heavy chain and have the same antigen affinity as normal antibodies consisting of light and heavy chains. Consequently camel antibodies are much smaller than, e.g., human antibodies, which allows them to penetrate dense tissues to reach the antigen, where larger proteins cannot. Moreover, the comparative simplicity, high affinity and specificity and the potential to reach and interact with active sites, camel's heavy chain antibodies present advantages over common antibodies in the design, production and application of clinically valuable compounds.

According to another preferred embodiment of the present invention the structural loop regions of a modular antibody or an immunoglobulins of camelid origin are modified, e.g. within a VHH, in the region of amino acids 7 to 19, amino acids 43 to 55, amino acids 68 to 76, amino acids 80 to 87 and amino acids 91 to 101, or within the terminal sequences, preferably within 6 amino acids from the C- or N-terminus of the antibody domain.

All numbering of the amino acid sequences of the immunoglobulins is according to the IMGT numbering scheme (IMGT, the international ImmunoGeneTics, Lefranc et al., 1999, Nucleic Acids Res. 27: 209-212).

The preferred method of producing the modular antibody according to the invention refers to engineering a modular antibody that is binding specifically to at least one first epitope, which comprises modifications in each of at least two sites or loops within a structural loop region, and determining the specific binding of said structural loop region to at least one second epitope in a screening process, wherein the unmodified structural loop region (non-CDR region) does not specifically bind to said at least one second epitope. Thus, an antibody or antigen-binding structure specific for a first antigen may be improved by adding another valency or specificity against a second antigen, which specificity may be identical, either targeting different epitopes or the same epitope, to increase valency or to obtain bi-, oligo- or multispecific molecules.

On the other hand it is preferred to make use of those modular antibodies that contain native structures interacting with effector molecules or immune cells, preferably to bind an effector ligand. Those native structures either remain unchanged or are modulated for an increased effector function. Binding sites for e.g. Fc receptors are described to be located in a CH2 and/or CH3 domain region, and may be mutagenized by well known techniques.

Preferred modular antibodies according to the invention are binding said individual antigens with a high affinity, in particular with a high on and/or a low off rate, or a high avidity of binding. The binding affinity of an antibody is usually characterized in terms of the concentration of the antibody, at which half of the antigen binding sites are occupied, known as the dissociation constant (Kd, or K_{D}). Usually a binder is considered a high affinity binder with a Kd<10⁻⁸ M, preferably a Kd<10⁻⁹ M, even more preferred is a Kd<10⁻¹⁰ M.

Yet, in a particularly preferred embodiment the individual antigen binding affinities are of medium affinity, e.g. with a Kd of less than 10⁻⁶ and up to 10⁻⁸ M, when the modular antibody according to the invention, which is crosslinking the targets of at least two binding sites, results in an affinity with a Kd<10⁻⁸ M of binding the target cell in sum.

Medium affinity binders may be provided according to the invention as well, preferably in conjunction with an affinity maturation process if necessary.

Affinity maturation is the process by which antibodies with increased affinity for antigen are produced. With structural changes of an antibody, including amino acid mutagenesis or as a consequence of somatic mutation in immunoglobulin gene segments, variants of a binding site to an antigen are produced and selected for greater affinities. Affinity matured modular antibodies may exhibit a several logfold greater affinity than a parent antibody. Single parent antibodies may be subject to affinity maturation. Alternatively pools of modular antibodies with similar binding affinity to the target antigen may be considered as parent structures that are varied to obtain affinity matured single antibodies or affinity matured pools of such antibodies.

The preferred affinity maturated variant of a modular antibody according to the invention exhibits at least a 10 fold increase in affinity of binding, preferably at least a 100 fold increase. The affinity maturation may be employed in the course of the selection campaigns employing respective libraries of parent molecules, either with modular antibodies having medium binding affinity to obtain the modular antibody of the invention having the specific target binding property of a binding affinity Kd<10⁻⁸ M. Alternatively, the affinity may be even more increased by affinity maturation of the modular antibody according to the invention to obtain the high values corresponding to a Kd of less than 10⁻⁹ M, preferably less than 10⁻¹⁰ M or even less than 10⁻¹¹ M, most preferred in the picomolar range.

The apoptotic effect of the modular antibody according to the invention has the advantage of a biological cytotoxic activity, which usually differs from any synthetic cytotoxic activity, e.g. as provided through a toxin that may be conjugated to an immunoglobulin structure. Toxins usually do not activate programmed cell death and the biological defence mechanism. Thus, the preferred apoptotic activity of the modular antibodies according to the invention is a biological apoptotic activity, leading to effective cytolysis.

Apoptosis is differentiated from the simple cell inhibition effect, where a substance is inhibiting cell growth, e.g. by binding to the receptor of a growth factor, thus blocking the growth factor function, or by inhibiting angiogenesis. Cytotoxicity through apoptosis is essentially considered as a programmed cell death , , and thus considered as a highly efficient way to immediately reduce the number of malignant cells. Cell growth inhibitors do not immediately kill cells, but only reduce the cell growth and proliferation, thus are considered to be less active for therapeutic purposes.

The modular antibody of the present invention may find use in a wide range of indications for antibody products. In one embodiment the modular antibody of the present invention is used for therapy or prophylaxis, e.g. as passive immunotherapy, for preparative, industrial or analytic use, as a diagnostic, an industrial compound or a research reagent, preferably a therapeutic. The modular antibody may find use in an antibody composition that is monoclonal or polyclonal. In a preferred embodiment, the modular antibodies of the present invention are used to capture or kill target cells that bear the target antigen, for example cancer cells that express the Lewis y and/ or the receptor targets.

For particular applications the modular antibody according to the invention is conjugated to a label or reporter molecule, selected from the group consisting of organic molecules, enzyme labels, radioactive labels, colored labels, fluorescent labels, chromogenic labels, luminescent labels, haptens, digoxigenin, biotin, metal complexes, metals, colloidal gold and mixtures thereof. Modified immunoglobulins conjugated to labels or reporter molecules may be used, for instance, in assay systems or diagnostic methods.

The modular antibody according to the invention may be conjugated to other molecules which allow the simple detection of said conjugate in, for instance, binding assays (e.g. ELISA) and binding studies.

In a preferred embodiment, a modular antibody is administered to a patient to treat a specific disorder. A "patient" for the purposes of the present invention includes humans and other animals, preferably mammals and most preferably humans. By "specific disorder" herein is meant a disorder that may be ameliorated by the administration of a pharmaceutical composition comprising a modular antibody of the present invention.

The modular antibody according to the invention is typically used to reduce the likelihood of metastasis developing, shrink tumor size, or slow tumor growth. It may be applied after surgery (adjuvant), before surgery (neo-adjuvant), or as the primary therapy (palliative). In one embodiment, a modular antibody according to the present invention is the only therapeutically active agent administered to a patient. Alternatively, the modular antibody according the present invention is administered in combination with one or more other therapeutic agents, including but not limited to cytotoxic agents, chemotherapeutic agents, cytokines, growth inhibitory agents, anti-hormonal agents, kinase inhibitors, anti-angiogenic agents, cardioprotectants, or other therapeutic agents. The modular antibody may be administered concomitantly with one or more other therapeutic regimens. For example, a modular antibody of the present invention may be administered to the patient along with chemotherapy, radiation therapy, or both chemotherapy and radiation therapy. Specifically the modular antibody according to the invention is used for neoadjuvant or adjuvant treatment to treat solid tumor disease conditions, which is either before, simultaneously or after concomitant therapy. Combination with standard treatment is particularly preferred, e.g. as second line treatment. Yet, the modular antibody according to the invention or the combination with standard therapy may as well be indicated as a first line treatment.

A combination therapy is particularly employing a standard regimen, e.g. as used for treating breast cancer, colorectal cancer, head and neck cancer or gastric cancer. This may include cyclophosphamide, doxorubicin, docetaxel, taxane, methotrexate and fluorouracil. Standard treatment of colorectal cancer typically involves the use of 5-fluorouracil (5-FU) or capecitabine (Xeloda), leucovorin (LV, Folinic Acid), oxaliplatin (Eloxatin), oxaliplatin or irinotecan.

In one embodiment, the modular antibody of the present invention may be administered in conjunction with one or more antibodies, which may or may not comprise a modular antibody of the present invention. In accordance with another embodiment of the invention, the modular antibody of the present invention and one or more other anti-cancer therapies is employed to treat cancer cells *ex vivo.* It is contemplated that such *ex vivo* treatment may be useful in bone marrow transplantation and particularly, autologous bone marrow transplantation. It is of course contemplated that the antibodies of the invention can be employed in combination with still other therapeutic techniques such as surgery.

A variety of other therapeutic agents may find use for administration with the modular antibody of the present invention. In one embodiment, the modular antibody is administered with an anti-angiogenic agent, which is a compound that blocks, or interferes to some degree, the development of blood vessels. The anti-angiogenic factor may, for instance, be a small molecule or a protein, for example an antibody, Fc fusion molecule, or cytokine, that binds to a growth factor or growth factor receptor involved in promoting angiogenesis. The preferred anti-angiogenic factor herein is an antibody that binds to Vascular Endothelial Growth Factor (VEGF). In an alternate embodiment, the modular antibody is administered with a therapeutic agent that induces or enhances adaptive immune response, for example an antibody that targets CTLA-4. In an alternate embodiment, the modified immunoglobulin is administered with a tyrosine kinase inhibitor, which is a molecule that inhibits to some extent tyrosine kinase activity of a tyrosine kinase. In an alternate embodiment, the modular antibody of the present invention is administered with a cytokine. By "cytokine" as used herein is meant a generic term for proteins released by one cell population that act on another cell as intercellular mediators including chemokines.

Pharmaceutical compositions are contemplated wherein modular antibodies of the present invention and one or more therapeutically active agents are formulated. Stable formulations of the modular antibodies of the present invention are prepared for storage by mixing said immunoglobulin having the desired degree of purity with optional pharmaceutically acceptable carriers, excipients or stabilizers, in the form of lyophilized formulations or aqueous solutions. The formulations to be used for in vivo administration are preferably sterile. This is readily accomplished by filtration through sterile filtration membranes or other methods. The modular antibody and other therapeutically active agents disclosed herein may also be formulated as immunoliposomes, and/or entrapped in microcapsules.

Administration of the pharmaceutical composition comprising a modular antibody of the present invention, preferably in the form of a sterile aqueous solution, may be done in a variety of ways, including, but not limited to, orally, subcutaneously, intravenously, intranasally, intraotically, transdermally, mucosal, topically (e.g., gels, salves, lotions, creams, etc.), intraperitoneally, intramuscularly, intrapulmonary (e.g., AERx^{™} inhalable technology commercially available from Aradigm, or Inhance^{™} pulmonary delivery system commercially available from Inhale Therapeutics), vaginally, parenterally, rectally, or intraocularly.

The invention also provides a method of producing a modular antibody according to the invention, which is composed of antibody modules, wherein at least two modules bear an antigen-binding site. The modules may be engineered and selected separately for the desired binding properties and then combined, preferably by fusion or by recombinant techniques. Alternatively, a modular antibody having a binding site for one of the glycoepitope or the receptor may be engineered to introduce a further antigen binding site without any further combination needs.

Engineering new antigen binding sites is preferably employing diversification of nucleic acid or amino acid sequences to provide libraries of variants, which may be selected for specific properties.

According to a preferred aspect modular antibodies are modified by a mutagenesis method to obtain a new binding site. The preferred mutagenesis refers to randomization techniques, where the amino acid sequence of a peptide or polypeptide is mutated in at least one position, thus a randomized sequence is obtained, which mediates antigen binding. For instance, specific antibody sequences are randomly modified to obtain a nucleic acid molecule coding for an immunoglobulin, immunoglobulin domain or a part thereof which comprises at least one nucleotide repeating unit, preferably within a structural loop coding region or within a terminal region, having the sequence 5'-NNS-3', 5'-NNN-3', 5'- NNB-3' or 5'- NNK-3'. In some embodiments the modified nucleic acid comprises nucleotide codons selected from the group of TMT, WMT, BMT, RMC, RMG, MRT, SRC, KMT, RST, YMT, MKC, RSA, RRC, NNK, NNN, NNS or any combination thereof (the coding is according to IUPAC).

The modification of the nucleic acid molecule may be performed by introducing synthetic oligonuleotides into a larger segment of nucleic acid or by de novo synthesis of a complete nucleic acid molecule. Synthesis of nucleic acid may be performed with tri-nucleotide building blocks which would reduce the number of nonsense sequence combinations if a subset of amino acids is to be encoded (e.g. Yanez et al. Nucleic Acids Res. (2004) 32:e158; Virnekas et al. Nucleic Acids Res. (1994) 22:5600-5607).

Another important aspect of the invention is that each potential binding domain remains physically associated with the particular DNA or RNA molecule which encodes it, and in addition, the fusion proteins oligomerize at the surface of a genetic package to present the binding polypeptide in the native and functional oligomeric structure. Once successful binding domains are identified, one may readily obtain the gene for expression, recombination or further engineering purposes. The form that this association takes is a "replicable genetic package", such as a virus, cell or spore which replicates and expresses the binding domain-encoding gene, and transports the binding domain to its outer surface. Another form is an in-vitro replicable genetic package such as ribosomes that link coding RNA with the translated protein. In ribosome display the genetic material is replicated by enzymatic amplification with polymerases.

Those cells or viruses or nucleic acid bearing the binding agents which recognize the target molecule are isolated and, if necessary, amplified. The genetic package preferably is M13 phage, and the protein includes the outer surface transport signal of the M13 gene III protein.

The preferred expression system for the fusion proteins is a non-suppressor host cell, which would be sensitive to a stop codon, such as an amber stop codon, and would thus stop translation thereafter. In the absence of such a stop codon such non-suppressor host cells, preferably E.coli, are preferably used. In the presence of such a stop codon supressor host cells would be used.

Preferably in the method of this invention the vector or plasmid of the genetic package is under tight control of the transcription regulatory element, and the culturing conditions are adjusted so that the amount or number of vector or phagemid particles displaying less than two copies of the fusion protein on the surface of the particle is less than about 20%. More preferably, the amount of vector or phagemid particles displaying less than two copies of the fusion protein is less than 10% the amount of particles displaying one or more copies of the fusion protein. Most preferably the amount is less than 1%.

The expression vector preferably used according to the invention is capable of expressing a binding polypeptide, and may be produced as follows: First a binding polypeptide gene library is synthesized by introducing a plurality of polynucleotides encoding different binding sequences. The plurality of polynucleotides may be synthesized in an appropriate amount to be joined in operable combination into a vector that can be propagated to express a fusion protein of said binding polypeptide. Alternatively the plurality of oligonucleotides can also be amplified by polymerase chain reaction to obtain enough material for expression. However, this would only be advantageous if the binding polypeptide would be encoded by a large polynucleotide sequence, e.g. longer than 200 base pairs or sometimes longer than 300 base pairs. Thus, a diverse synthetic library is preferably formed, ready for selecting from said diverse library at least one expression vector capable of producing binding polypeptides having the desired preselected function and binding property, such as specificity.

The randomly modified nucleic acid molecule may comprise the above identified repeating units, which code for all known naturally occurring amino acids or a subset thereof. Those libraries that contain modified sequences wherein a specific subset of amino acids are used for modification purposes are called "focused" libraries. The member of such libraries have an increased probability of an amino acid of such a subset at the modified position, which is at least two times higher than usual, preferably at least 3 times or even at least 4 times higher. Such libraries have also a limited or lower number of library members, so that the number of actual library members reaches the number of theoretical library members. In some cases the number of library members of a focused library is not less than 10³ times the theoretical number, preferably not less than 10² times, most preferably not less than 10 times.

Various alternatives are available for the manufacture of a randomized library. It is possible to produce the DNA by a completely synthetic approach, in which the sequence is divided into overlapping fragments which are subsequently prepared as synthetic oligonucleotides. These oligonucleotides are mixed together, and annealed to each other by first heating to ca. 100°C and then slowly cooling down to ambient temperature. After this annealing step, the synthetically assembled gene can be either cloned directly, or it can be amplified by PCR prior to cloning.

Alternatively, other methods for site directed mutagenesis can be employed for generation of the library insert, such as the Kunkel method (Kunkel TA. Rapid and efficient site-specific mutagenesis without phenotypic selection. Proc Natl Acad Sci U S A. 1985 Jan;82(2):488-92) or the Dpnl method (Weiner MP, Costa GL, Schoettlin W, Cline J, Mathur E, Bauer JC. Site-directed mutagenesis of double-stranded DNA by the polymerase chain reaction. Gene. 1994 Dec 30;151(1-2):119-23.).

For various purposes, it may be advantageous to introduce silent mutations into the sequence encoding the library insert. For example, restriction sites can be introduced which facilitate cloning or modular exchange of parts of the sequence. Another example for the introduction of silent mutations is the ability to "mark" libraries, that means to give them a specific codon at a selected position, allowing them (or selected clones derived from them) e.g. to be recognized during subsequent steps, in which for example different libraries with different characteristics can be mixed together and used as a mixture in the panning procedure.

The method according to the invention can provide a library containing at least 10² independent clones expressing functional modular antibody domains. According to the invention it is also provided a pool of preselected independent clones, which is e.g. affinity maturated, which pool comprises preferably at least 10, more preferably at least 100, more preferably at least 1000, more preferably at least 10000, even more than 100000 independent clones. Those libraries, which contain the preselected pools, are preferred sources to select the high affinity modular antibodies according to the invention.

Usually the libraries as used according to the invention contain variants of the modular antibody, resulting from mutagenesis or randomization techniques. These variants include inactive or non-functional antibodies. Thus, it is preferred that any such libraries be screened with the appropriate assay for determining the functional effect. Preferred libraries, according to the invention, comprise at least 10² variants of modular antibodies, more preferred at least 103, more preferred at least 10⁴, more preferred at least 10⁵, more preferred at least 10⁶, more preferred at least 10⁷, more preferred at least 10⁸, more preferred at least 10⁹, more preferred at least 10¹⁰, more preferred at least 10¹¹, up to 10¹² variants or higher to provide a highly diverse repertoire of modular antibodies for selecting the best suitable binders. Any such synthetic libraries may be generated using mutagenesis methods as disclosed herein.

Libraries as used according to the invention preferably comprise at least 10² library members, more preferred at least 10³, more preferred at least 10⁴, more preferred at least 10⁵, more preferred at least 10⁶ library members, more preferred at least 10⁷, more preferred at least 10⁸, more preferred at least 10⁹, more preferred at least 10¹⁰, more preferred at least 10¹¹, up to 10¹² members of a library, preferably derived from a parent molecule, which is a functional modular antibody as a scaffold containing at least one specific function or binding moiety, and derivatives thereof to engineer a new binding site apart from the original, functional binding region of said parent moiety.

A library as used according to the invention may be designed as a dedicated library based on specific modular antibody formats, preferably selected from the group consisting of a VH library, VHH library, Vkappa library, Vlambda library, Fab library, a CH1/CL library, an Fc library and a CH3 library. Libraries characterized by the content of composite molecules containing more than one antibody domains, such as an IgG library or Fc library are specially preferred. Other preferred libraries are those containing T-cell receptors, forming T-cell receptor libraries. Further preferred libraries are epitope libraries, wherein the fusion protein comprises a molecule with a variant of an epitope, also enabling the selection of competitive molecules having similar binding function, but different functionality.

Preferably the library is a yeast library and the yeast host cell exhibits at the surface of the cell the oligomers with the biological activity. The yeast host cell is preferably selected from the genera Saccharomyces, Pichia, Hansenula, Schizisaccharomyces, Kluyveromyces, Yarrowia and Candida. Most preferred, the host cell is *Saccharomyces cerevisiae.*

As is well-known in the art, there is a variety of display and selection technologies that may be used for the identification and isolation of proteins with certain binding characteristics and affinities, including, for example, display technologies such as cellular and non-cellular, in particular mobilized display systems. Among the cellular systems the phage display, virus display, yeast or other eukaryotic cell display, such as mammalian or insect cell display, may be used. Mobilized systems are relating to display systems in the soluble form, such as in vitro display systems, among them ribosome display, mRNA display or nucleic acid display.

Methods for production and screening of antibody variants are well-known in the art. General methods for antibody molecular biology, expression, purification, and screening are described in Antibody Engineering, edited by Duebel & Kontermann, Springer-Verlag, Heidelberg, 2001; and Hayhurst & Georgiou, 2001, Curr Opin Chem Biol 5:683-689; Maynard & Georgiou, 2000, Annu Rev Biomed Eng 2:339-76.

Specifically modular antibodies may be designed by producing respective variants and screening for specific properties.

The ADCC of a modular antibody according to the invention may be determined using typical assays employ target cells, like Ramos cells, incubated with serially diluted antibody prior to the addition of freshly isolated effector cells. The ADCC assay is then further incubated for several hours and % cytotoxicity detected. Usually the target: effector ratio is about 1:16, but may be 1:1 up to 1:50.

The CDC of a modular antibody according to the invention may be determined employing the mechanism of killing cells, in which antibody bound to the target cell surface fixes complement, which results in assembly of the membrane attack complex that punches holes in the target cell membrane resulting in subsequent cell lysis. The commonly used CDC assay follows the same procedure as for ADCC determination, however, with complement containing serum instead of effector cells.

A cytotoxic activity as determined by either of an ADCC or CDC assay may be shown for a modular antibody according to the invention, if there is a significant increase in the percentage of cytolysis as compared to a control. The cytotoxic activity related to ADCC or CDC is preferably measured as the absolute percentage increase, which is preferably higher than 5%, more preferably higher than 10%, even more preferred higher than 20%.

The antibody-dependent cellular phagocytosis, ADCP sometimes called ADPC, is usually investigated side by side with cytolysis of cultured human cells. Phagocytosis by phagocytes, usually human monocytes or monocyte-derived macrophages, as mediated by an antibody can be determined as follows. Purified monocytes may be cultured with cytokines to enhance expression of FcyRs or to induce differentiation into macrophages. ADCP and ADCC assays are then performed with target cells. Phagocytosis is determined as the percentage of positive cells measured by flow cytometry. The positive ADCP activity is proven with a significant uptake of the antibody-antigen complex by the phagocytes. The cytotoxic activity related to ADCP is preferably measured as the absolute percentage uptake of the antibody-antigen complex by the phagocytes, which is preferably higher than 5%, more preferably higher than 10%, even more preferred higher than 20%.

In a typical assay PBMC or monoycytes or monocyte derived macrophages are resuspended in RF2 medium (RPMI 1640 supplemented with 2% FCS) in 96-well plates at a concentration of 1 x 10⁵ viable cells in 100 ml/well. Appropriate target cells, expressing the target antigen, e.g. Her2/neu antigen and SKBR3 cells, are stained with PKH2 green fluorescence dye. Subsequently 1 x 10⁴ PKH2-labeled target cells and an Her2 specific (IgG1) antibody (or modular antibody) or mouse IgG1 isotype control (or modular antibody control) are added to the well of PBMC's in different concentrations (e.g. 1-100 pg/ml) and incubated in a final volume of 200 ml at 37°C for 24 h. Following the incubation, PBMCs or monoycytes or monocyte derived macrophages and target cells are harvested with EDTA-PBS and transferred to 96-well V-bottomed plates. The plates are centrifuged and the supernatant is aspirated. Cells are counterstained with a 100-ml mixture of RPE-conjugated anti-CD11b, anti-CD14, and human IgG, mixed and incubated for 60 min on ice. The cells are washed and fixed with 2% formaldehyde-PBS. Two-color flow cytometric analysis is performed with e.g. a FACS Calibur under optimal gating. PKH2-labeled target cells (green) are detected in the FL-1 channel (emission wavelength, 530 nm) and RPE-labeled PBMC or monoycytes or monocyte derived macrophages (red) are detected in the FL-2 channel (emission wavelength, 575 nm). Residual target cells are defined as cells that are PKH2⁺/RPE⁻ Dual-labeled cells (PKH2⁺/RPE⁻) are considered to represent phagocytosis of targets by PBMC or monoycytes or monocyte derived macrophages. Phagocytosis of target cells is calculated with the following equation: percent phagocytosis = 100 x [(percent dual positive)/ (percent dual positive + percent residual targets)]. All tests are usually performed in duplicate or triplicate and the results are expressed as mean 6 SD.

In a preferred embodiment, antibody variants are screened using one or more cell-based or *in vivo* assays. For such assays, purified or unpurified modified immunoglobulins are typically added exogenously such that cells are exposed to individual immunoglobulins or pools of immunoglobulins belonging to a library. These assays are typically, but not always, based on the function of the immunoglobulin; that is, the ability of the antibody to bind to its target and mediate some biochemical event, for example effector function, ligand/receptor binding inhibition, apoptosis, and the like. Such assays often involve monitoring the response of cells to the antibody, for example cell survival, cell death, change in cellular morphology, or transcriptional activation such as cellular expression of a natural gene or reporter gene. For example, such assays may measure the ability of antibody variants to elicit ADCC, ADCP, CDC or apoptotic activity. For some assays additional cells or components, that is in addition to the target cells, may need to be added, for example example serum complement, or effector cells such as peripheral blood monocytes (PBMCs), NK cells, macrophages, and the like. Such additional cells may be from any organism, preferably humans, mice, rat, rabbit, and monkey. Modular antibodies may cause apoptosis of certain cell lines expressing the target, or they may mediate attack on target cells by immune cells which have been added to the assay. Methods for monitoring cell death or viability are known in the art, and include the use of dyes, immunochemical, cytochemical, and radioactive reagents. For example, caspase staining assays may enable apoptosis to be measured, and uptake or release of radioactive substrates or fluorescent dyes such as alamar blue may enable cell growth or activation to be monitored.

In a preferred embodiment, the DELFIART EuTDA-based cytotoxicity assay (Perkin Elmer, MA) may be used. Alternatively, dead or damaged target cells may be monitored by measuring the release of one or more natural intracellular components, for example lactate dehydrogenase.

Transcriptional activation may also serve as a method for assaying function in cell-based assays. In this case, response may be monitored by assaying for natural genes or immunoglobulins which may be upregulated, for example the release of certain interleukins may be measured, or alternatively readout may be via a reporter construct. Cell-based assays may also involve the measure of morphological changes of cells as a response to the presence of modular antibodies. Cell types for such assays may be prokaryotic or eukaryotic, and a variety of cell lines that are known in the art may be employed. Alternatively, cell-based screens are performed using cells that have been transformed or transfected with nucleic acids encoding the variants. That is, antibody variants are not added exogenously to the cells. For example, in one embodiment, the cell-based screen utilizes cell surface display. A fusion partner can be employed that enables display of modified immunoglobulins on the surface of cells (Witrrup, 2001, Curr Opin Biotechnol, 12:395-399).

In a preferred embodiment, the immunogenicity of the modular antibodies may be determined experimentally using one or more cell-based assays. In a preferred embodiment, *ex vivo* T-cell activation assays are used to experimentally quantitate immunogenicity. In this method, antigen presenting cells and naive T cells from matched donors are challenged with a peptide or whole antibody of interest one or more times. Then, T cell activation can be detected using a number of methods, for example by monitoring production of cytokines or measuring uptake of tritiated thymidine. In the most preferred embodiment, interferon gamma production is monitored using Elispot assays.

The biological properties of the modular antibody according to the invention may be characterized *ex vivo* in cell, tissue, and whole organism experiments. As is known in the art, drugs are often tested *in vivo* in animals, including but not limited to mice, rats, rabbits, dogs, cats, pigs, and monkeys, in order to measure a drug's efficacy for treatment against a disease or disease model, or to measure a drug's pharmacokinetics, pharmacodynamics, toxicity, and other properties. The animals may be referred to as disease models. Therapeutics are often tested in mice, including but not limited to nude mice, SCID mice, xenograft mice, and transgenic mice (including knockins and knockouts). Such experimentation may provide meaningful data for determination of the potential of the antibody to be used as a therapeutic with the appropriate half-life, effector function, apoptotic activity, cytotoxic or cytolytic activity. Any organism, preferably mammals, may be used for testing. For example because of their genetic similarity to humans, primates, monkeys can be suitable therapeutic models, and thus may be used to test the efficacy, toxicity, pharmacokinetics, pharmacodynamics, half-life, or other property of the modular antibody according to the invention. Tests of the substances in humans are ultimately required for approval as drugs, and thus of course these experiments are contemplated. Thus the modular antibodies of the present invention may be tested in humans to determine their therapeutic efficacy, toxicity, immunogenicity, pharmacokinetics, and/or other clinical properties. Especially those modular antibodies according to the invention that bind to single cell or a cellular complex through at least two binding motifs, preferably binding of at least three structures cross-linking target cells, would be considered effective in effector activity or preapoptotic or apoptotic activity upon cell targeting and crosslinking. Multivalent binding provides a relatively large association of binding partners, also called cross-linking, which is a prerequisite for apoptosis and cell death.

The foregoing description will be more fully understood with reference to the following examples. Such examples are, however, merely representative of methods of practicing one or more embodiments of the present invention and should not be read as limiting the scope of invention.

### Examples

### Example 1: Construction of antigen binding Fc (Fcab) binding to Her2 and EGFR

The identification of the Her-2 specific Fcab clone H561-4 is described previously (WO2009132876A1). Briefly, populations of antigen specific Fcabs expressed on the surface of yeast cells are enriched from large yeast Fcab libraries by repeated rounds of selections in a high speed cell sorter. In an analogous process, Fcab clones specific for binding to the extracellular domain of another receptor are enriched. Individual clones from enriched populations are screened for antigen binding and the best clones are expressed as soluble proteins in mammalian cells for further characterization.

**Table 1: Capital letters denote non-CDR loop amino acids; small letters indicate framework amino acids flanking the loop sequences.**

| Specificity | Fcab | AB_loop | EF loop |
|---|---|---|---|
| none | Wild type | deLTKNQvsl (SEQ ID No. 12) | tvDKSRWQQgn (SEQ ID No. 13) |
| Her-2 | H561-4 | deFFTYWvsl (SEQ lD No. 14) | tvDRRRWTAgn (SEQ lD No. 15) |

### Expression and purification of antigen specific clones in mammalian cells:

Clones selected as described above with characteristics as described above are cloned into a mammalian expression vector such as pCEP4 (Invitrogen) as a Kpnl/BamHl fragment. The resulting Fcab expression plasmids contain an open reading frame comprising the last 6 amino acids of the hinge region, the CH2 domain and the antigen binding CH3 domain. To facilitate further cloning steps a Xhol restriction site is introduced at the CH2/CH3 domain junction. Highly purified plasmid DNA (Qiagen) is used to transiently transfect HEK293 freestyle cells with Freestyle™ MAX Reagent as recommended by the manufacturer (Invitrogen). On day 5 post transfection, cell supernatants are cleared from cell debris by centrifugation and filtration through a 0.2µM Stericup filter (Millipore). Alternatively, HEK293 freestyle cells or CHO cells are transfected with expression plasmids containing genes for antibiotics resistance such as neomycin or puromycin. The transfected cells are cultivated in the presence of the antibiotics resulting in specific survival of cell clones which stably express the antibiotics resistance gene together with the antigen specific Fc fragment. Such stable transfectants consistently secrete the protein of interest over long time periods. The antigen specific Fcabs are purified from cell supernatants by Protein A immuno-affinity chromatography. Bound Fcabs are eluted from Protein A by washing the column with glycine buffer (pH=2.9-4.0), followed by dialysis against PBS (pH=6.8). The purity of the Fcabs is determined by non-reducing SDS-PAGE analysis and potential aggregates are detected by size-exclusion HPLC using a Zorbax GF250 column and PBS as running buffer.

### Example 2: Binding affinities of Her-2 specific Fcabs

The binding affinity of human Her-2 specific Fcab H561-4 is determined by surface plasmon resonance (SPR) assays in a Biacore instrument. CM-5 chips are coated with increasing concentrations of recombinant soluble HER-2 protein. Afterwards, increasing concentrations of Fcab H561-4 (0.8-25µg/ml) are injected on each coated chip until binding equilibrium to HER-2 is reached. Then, buffer is injected to measure the off-rate of the binding reaction. The binding affinity (K_{D}) is calculated using the BiaEval software using a 1:1 stochiometry model. These experiments indicate that Fcab H561-4 has a binding affinity for recombinant HER-2 of 7.5nM (figure 1, right panel). Alternatively, binding of Fcab H561-4 to HER-2 expressed on human tumor cells is determined. A constant cell number of the human breast cancer cell line SKBR3 (1x10⁵ cells) is incubated with increasing amounts of Fcab H561-4 in FACS buffer (PBS containing 0.1 % bovine serum albumin) for 60 minutes on ice. Unbound Fcab is removed by two washing steps in FACS buffer. Cell bound Fcab is detected by incubation of the cells with polyclonal anti-human IgG antibodies coupled to phycoerythrin (SIGMA) for 30 minutes on ice as recommended by the manufacturer. Again, unbound detection antibodies are removed by two washing steps as above. Fcab binding is enumerated by flow cytometry by plotting the mean fluorescence intensity against the Fcab concentrations (figure 1, left panel). These experiments indicate an apparent EC₅₀ binding for Fcab H561-4 of 2nM which is in good agreement with the SPR data.

### Example 3: Engineering the Lewis y/HER2 bispecific monoclonal antibody (mAb²)

The monoclonal antibody VL311 recognizes the glyco-epitope Lewis Y (EP528767A1). The monoclonal antibody BW835 is directed against a different carbohydrate epitope called Thomsen-Friedenreich (Hanisch FG, Stadie T, Boßlet K. Cancer Res. 1995; 55:4036-40). The gene sequences encoding the VH domains of mAbs VL311 and BW835 are synthesized by a commercial source as KpnI/NheI fragments. The corresponding VL sequences are synthesized as KpnI/KasI fragments. The DNA fragments are ligated into two mammalian expression plasmids based on the pCEP4 vector (Invitrogen). One of the pCEP4 plasmids contains the complete heavy chain gene of the OKT3 monoclonal antibody (human IgG1 isotype) (Adair JR, Athwal DS, Bodmer MW, Bright SM, Collins AM, Pulito VL, Rao PE, Reedman R, Rothermel AL, Xu D, et al. Hum Antibodies Hybridomas. 1994;5(1-2):41-7) cloned as a KpnI/BamHI fragment. The second pCEP4 expression vector encodes the complete light chain gene of the OKT3 antibody cloned as a KpnI/BamHI fragment. To facilitate cloning of individual antibody domains derived from other antibodies, unique restriction enzyme cleavage sites were introduced in frame at the junctions between the OKT3 VH and CH1 domains (Nhel), between VL and CL (KasI) and between the CH2 and CH3 domains of the heavy chain (Xhol). Therefore, replacement of the KpnI/NheI OKT3 VH gene segment with the VH gene fragments of the VL311 and BW835 antibodies regenerate complete human IgG1 heavy chains. Similarly, the OKT3 VL gene segment can be excised as a KpnI/KasI fragment and replaced with the VL segments of VL311 and BW835 resulting in regeneration of a complete light chain.

For the cloning of VL311 and BW835 mAb² expression constructs, a XhoI/BamHI fragment containing the wild type CH3 domain was replaced with the CH3 domain of Fcab H561-4.

### Example 4: direct tumor cell killing by Lewis y/ErbB mAb² and TF/ErbB mAb²

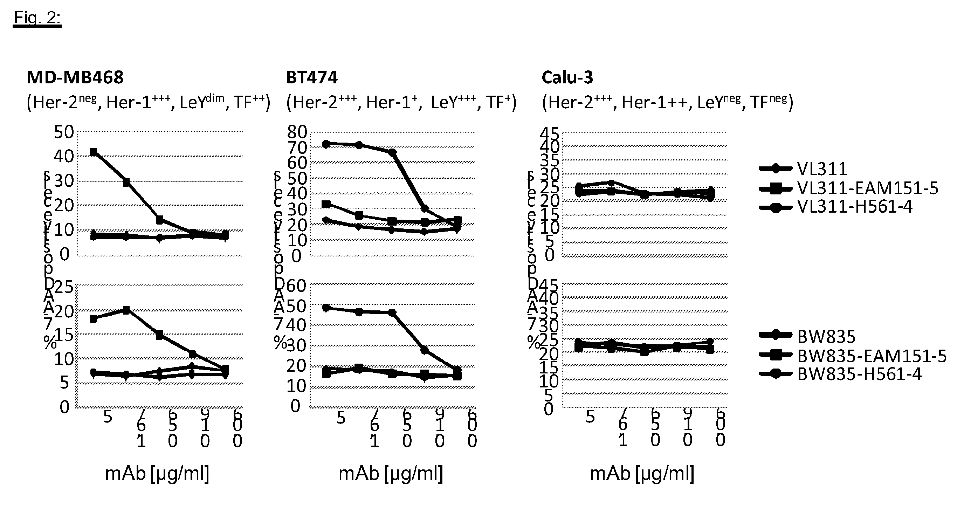

In order to assess the effect of antibodies on tumor cell growth, three human tumor cell lines expressing different levels of HER2, HER1, Lewis Y and the Thomsen-Friedenreich (TF) antigen are used (BT474, Calu-3 and MD-MBA468, obtained from LGC Standards). 1x10⁵ cells are seeded in 96well microtiter plates and incubated at 37°C with increasing concentrations of the parental antibodies VL311 and BW835. Parallel cultures receive mAb² thereof containing an additional HER2 binding site in the CH3 domain (VL311-H561-4, BW835-H561-4) or an Her-1 binding site (VL311-EAM151-5, BW835-EAM151-5). After a 4 hour incubation period, cells are harvested by trypsinization, washed and incubated with 7-amino-actinomycin D (7-AAD). This compound binds to DNA in dying cells and, due to its fluorescent properties can be detected by flow cytometry. Thus, enumeration of fluorescent cells is a direct measure for dying cells. The data demonstrate that both parental antibodies have no effect on the growth of the three cell lines. By contrast, HER2 binding site containing mAb² are able to kill BT474 cells which express HER2, LeY and TF antigens while having no effect on MD-MBA468 cells which do not express HER2 but are positive for both glycoepitopes. In contrast, both mAb² with the HER1 binding site are able to elicit cell death in MD-MBA468 cells which express high levels of HER1 and both Lewis Y and TF antigens. No killing of BT474 by BW835-EAM151-5 is seen probably due to low expression of HER-1 and TF on these cells. Low killing activity is seen with VL311-EAM151-5 in BT474 cells, presumably due to its high expression levels of Lewis Y. None of the mAb² is able to kill Calu-3 cells which do express both ErbB family members but are devoid of the two glyco-epitopes under study (figure 2). Therefore, mAb² specific cell killing depends on the presence of both antigens on the tumor cell surface.

### Example 5: Mechanism of mAb² induced cell death

To determine, if Fcab H561-4 itself is responsible for the killing effect HCC1954 cells (HER2⁺⁺⁺, LeY⁺) are incubated with 18.5nM Fcab H561-4 alone. To further determine, if the way how HER2 and the Lewis Y antigen are engaged by antibodies plays a role for cell death induction, cells are treated with 6.25nM antibodies alone or in combinations as shown in figure 3. After a 4 hour incubation period at 37°C cells are harvested by trypsinization and washed. Dying cells are enumerated by determination of 7-AAD positive cells as described above. The data indicate that Fcab H561-4 alone has no effect on cell viability indicating that simultaneous binding of HER2 and Lewis Y is necessary for cell death induction. In addition, the mixture of VL311 and Fcab H561-4 or the mixture of VL311 and trastuzumab (trade name Herceptin, Genentech, a clinically approved HER-2 antibody) does not lead to any induction of cell death in contrast to mAb² VL311-H561-4 which induces a robust killing response. This data demonstrate that the modality of simultaneous engagement of HER-2 and Lewis Y determines if HCC1954 cells will be killed or not. Co-crosslinking of HER2 and LewisY by a single molecular entity, such as the mAb², provides the necessary signal for inducing cell death.

Apoptosis is a normal physiologic process which occurs during embryonic development as well as in maintenance of tissue homeostasis. The apoptotic program is characterized by certain morphologic features, including loss of plasma membrane asymmetry and attachment, condensation of the cytoplasm and nucleus, and internucleosomal cleavage of DNA. Loss of plasma membrane is one of the earliest features. In apoptotic cells, the membrane phospholipid phosphatidylserine (PS) is translocated from the inner to the outer leaflet of the plasma membrane, thereby exposing PS to the external cellular environment. Annexin V is a 35-36 kDa Ca2+ dependent phospholipid-binding protein that has a high affinity for PS, and binds to cells with exposed PS. Annexin V may be conjugated to fluorochromes including FITC.

This format retains its high affinity for PS and thus serves as a sensitive probe for flow cytometric analysis of cells that are undergoing apoptosis. Since externalization of PS occurs in the earlier stages of apoptosis, FITC Annexin V staining can identify apoptosis at an earlier stage than assays based on nuclear changes such as DNA fragmentation, a process which results from the activation of endonucleases during the apoptotic program.

To determine if the mechanism by which the mAb² proteins kill cells involves apoptosis, SKBR3 cells which express HER-2 and Lewis Y are incubated with increasing concentrations of parental VL311 mAb or mAb² VL311-H561-4 for 24 hours at 37°C. Afterwards, cells are harvested by trypsinization and washed. One cell aliquot was probed for the presence of Annexin V positivity using the FITC Annexin V Apoptosis Detection Kit I (Beckton Dickinson). Another cell aliquot was taken for detection of chromosomal DNA fragmentation (APO-Direct Kit, Beckton Dickinson). The underlying principle in this kit is often referred to as "TUNEL" (dUTP nick end labeling) assay. Shortly, the enzyme terminal deoxynucleotidyltransferase (TdT) catalyzes addition of bromolated deoxyuridine triphosphates (Br-dUTP) to the 3'-hydroxyl termini of double- and single-stranded DNA. After incorporation, these sites are identified by flow cytometric means by staining the cells with a FITC-labeled anti-BrdU monoclonal antibody.

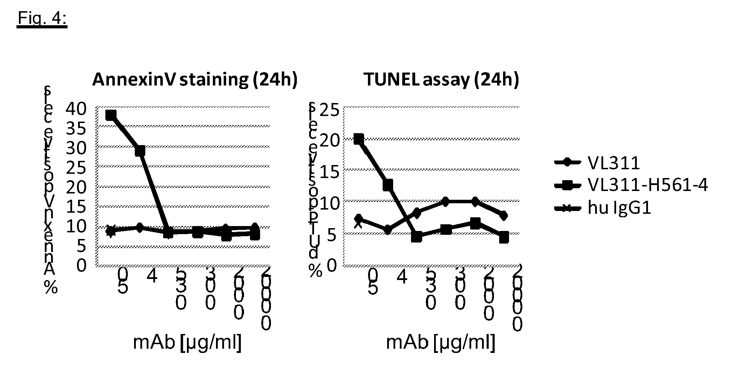

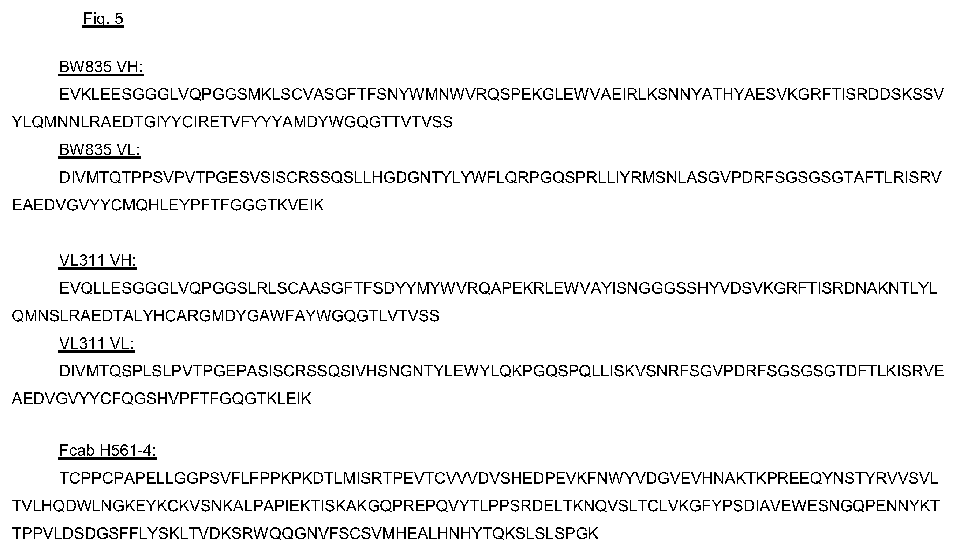

Both kits are used as recommended by the manufacturer. The data shown in figure 4 demonstrate that only mAb² VL311-H561-4, but not the parental antibody VL311 induces the appearance of Annexin V and dUTP positive cells indicative of early and later stages of apoptosis. Therefore, incubation of tumor cells with mAb² VL311-H561-4 kills cells by an apoptotic mechanism.

## Claims

1. Modular antibody having at least two specificities to crosslink a glycoepitope and a receptor of the erbB class on a tumor cell, which antibody is essentially free of effector function in the tumor cell based assay.

2. Modular antibody according to claim 1, wherein a first specificity is directed to a glycoepitope of an antigen selected from the group consisting of Lewis x-, Lewis b- and Lewis y-structures, Globo H-structures, KH1, Tn antigen, TF antigen and carbohydrate structures of Mucins, CD44, glycolipids and glycosphingolipids, such as Gg3, Gb3, GD3, GD2, Gb5, Gm1, Gm2, sialyltetraosylceramide.

3. Modular antibody according to any of claims 1 or 2, wherein a second specificity is directed to an erbB receptor tyrosine kinase selected from the group consisting of EGFR, HER2, HER3 and HER4.

4. Modular antibody according to any of claims 1 to 3, which contains a binding site having a randomized antibody sequence.

5. Modular antibody according to any one of claims 1 to 4, which contains a binding site within a structural loop region.

6. Modular antibody according to any one of claims 1 to 5, wherein said antibody is an oligomer of modular antibody domains.

7. Modular antibody according to claim 6, wherein said oligomer comprises immunoglobulin domains selected from the group consisting of VH/VL, CH1/CL, CH2/CH2, CH3/CH3, Fc, Fab and scFv.

8. Modular antibody according to any one of claims 1 to 7, wherein said antibody is a bispecific full-length immunoglobulin or Fcab.

9. Modular antibody according to any of claims 1 to 8, which simultaneously binds to both, the glycoepitope and said receptor on a tumor cell.

10. Modular antibody according to any of claims 1 to 9, which binds to the glycoepitope and said receptor on a tumor cell by at least three binding sites.

11. Modular antibody according to any of claims 1 to 10, which binds to said tumor cell with a Kd<10⁻⁸M.

12. Modular antibody according to any of claims 1 to 11, for the treatment of a patient suffering from a solid tumor, which tumor expresses a receptor of the erbB class and an aberrant glycosylation.

13. Modular antibody according to claim 12, for the treatment of breast cancer, colorectal cancer, head and neck cancer or gastric cancer.

14. Modular antibody according to claim 12 or 13, for the treatment of immunocompromised patients, preferably in combination with chemotherapy or radiotherapy.

15. Method of preparing a modular antibody according to any of claims 1 to 11, comprising
a. fusing or recombining the following components
(i) a modular antibody with a specificity to bind at least a glycoepitope and
(ii) a modular antibody with a specificity to bind at least a receptor of the erbB class,
to obtain a modular antibody with at least both specificities, and
b. determining the cytolysis of said tumor cell in the absence of NK cells.

## Amended claims

### Amended claims in accordance with Rule 137(2) EPC.

**1.** Modular antibody having at least two specificities to crosslink a glycoepitope and a receptor of the erbB class on a tumor cell, which antibody has apoptotic activity effecting cytolysis independent of NK cells and essentially free of effector function determined in the tumor cell based assay, wherein the modular antibody binds to the tumor cell expressing the glycoepitope and the receptor.

**2.** Modular antibody according to claim 1, wherein a first specificity is directed to a glycoepitope of an antigen selected from the group consisting of Lewis x-, Lewis b- and Lewis y-structures, Globo H-structures, KH1, Tn antigen, TF antigen and carbohydrate structures of Mucins, CD44, glycolipids and glycosphingolipids, such as Gg3, Gb3, GD3, GD2, Gb5, Gm1, Gm2, sialyltetraosylceramide.

**3.** Modular antibody according to any of claims 1 or 2, wherein a second specificity is directed to an erbB receptor tyrosine kinase selected from the group consisting of EGFR, HER2, HER3 and HER4.

**4.** Modular antibody according to any of claims 1 to 3, which contains a binding site having a randomized antibody sequence produced through mutagenesis of a nucleotide or amino acid sequence.

**5.** Modular antibody according to any one of claims 1 to 4, which contains a binding site within a structural loop region.

**6.** Modular antibody according to any one of claims 1 to 5, wherein said antibody is an oligomer of modular antibody domains.

**7.** Modular antibody according to claim 6, wherein said oligomer comprises immunoglobulin domains selected from the group consisting of VH/VL, CH1/CL, CH2/CH2, CH3/CH3, Fc, Fab and scFv.

**8.** Modular antibody according to any one of claims 1 to 7, wherein said antibody is a bispecific full-length immunoglobulin or Fcab.

**9.** Modular antibody according to any of claims 1 to 8, which additionally has cytotoxic activity associated with ADCC and/ or CDC activity.

**10.** Modular antibody according to any of claims 1 to 9, which simultaneously binds to both, the glycoepitope and said receptor on a tumor cell.

**11.** Modular antibody according to any of claims 1 to 10, which binds to the glycoepitope and said receptor on a tumor cell by at least three binding sites.

**12.** Modular antibody according to any of claims 1 to 11, which binds to said tumor cell with a Kd<10⁻⁸M.

**13.** Modular antibody according to any of claims 1 to 12, for the treatment of a patient suffering from a solid tumor, which tumor expresses a receptor of the erbB class and an aberrant glycosylation.

**14.** Modular antibody according to claim 13, for the treatment of breast cancer, colorectal cancer, head and neck cancer or gastric cancer.

**15.** Modular antibody according to claim 13 or 14, for the treatment of immunocompromised patients, preferably in combination with chemotherapy or radiotherapy.

**16.** Method of preparing a modular antibody according to any of claims 1 to 12, comprising
a. fusing or recombining the following components
(i) a modular antibody with a specificity to bind at least a glycoepitope and
(ii) a modular antibody with a specificity to bind at least a receptor of the erbB class,
to obtain a modular antibody with at least both specificities, and
b. determining the cytolysis of said tumor cell in the absence of NK cells.
